(19) Europäisches Patentamt / European Patent Office / Office européen des brevets

(11) **EP 1 560 815 B1**

(12) **EUROPÄISCHE PATENTSCHRIFT**

(45) Veröffentlichungstag und Bekanntmachung des Hinweises auf die Patenterteilung:
**12.03.2008 Patentblatt 2008/11**

(21) Anmeldenummer: **03810401.4**

(22) Anmeldetag: **18.10.2003**

(51) Int Cl.:
***C07D 239/28*** *(2006.01)* ***C07D 405/12*** *(2006.01)*
***C07D 401/12*** *(2006.01)* ***C07D 413/14*** *(2006.01)*
***C07D 405/14*** *(2006.01)* ***C07D 413/12*** *(2006.01)*
***A61K 31/505*** *(2006.01)* ***A61K 31/506*** *(2006.01)*
***A61P 19/00*** *(2006.01)*

(86) Internationale Anmeldenummer:
**PCT/EP2003/011515**

(87) Internationale Veröffentlichungsnummer:
**WO 2004/041788 (21.05.2004 Gazette 2004/21)**

(54) **NEUE PYRIMIDIN-4,6-DICARBONS UREDIAMIDE ZUR SELEKTIVEN INHIBIERUNG VON KOLLAGENASEN**

NOVEL PYRIMIDINE-4,6-DICARBOXAMIDES FOR THE SELECTIVE INHIBITION OF COLLAGENASES

NOUVEAUX DIAMIDES D'ACIDE PYRIMIDINE-4,6-DICARBOXYLIQUE POUR L'INHIBITION SELECTIVE DE COLLAGENASES

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **02.11.2002 DE 10251019**
**20.11.2002 DE 10254092**

(43) Veröffentlichungstag der Anmeldung:
**10.08.2005 Patentblatt 2005/32**

(73) Patentinhaber: **Sanofi-Aventis Deutschland GmbH**
**65929 Frankfurt am Main (DE)**

(72) Erfinder:
- **KLINGLER, Otmar**
**63110 Rodgau (DE)**
- **KIRSCH, Reinhard**
**38100 Braunschweig (DE)**
- **HABERMANN, Jörg**
**I-00040 Ariccia (IT)**
- **WEITHMANN, Klaus-Ulrich**
**65719 Hofheim (DE)**
- **ENGEL, Christian**
**60316 Frankfurt (DE)**
- **PIRARD, Bernard**
**Sanofi-Aventis Deutschland GmbH**
**65926 Frankfurt am Main (DE)**

(56) Entgegenhaltungen:
**EP-A- 0 418 797** **WO-A-02/064568**
**WO-A-02/064571** **WO-A-03/049738**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 560 815 B1

Printed by Jouve, 75001 PARIS (FR)

## Beschreibung

Die Erfindung betrifft neue Pyrimidin-4,6-dicarbon-säurediamide und deren Verwendung zur selektiven Inhibition der Kollagenase (MMP 13). Die Pyrimidin-4,6-dicarbonsäurediamide können daher zur Behandlung degenerativer Gelenkerkrankungen eingesetzt werden.

Es ist bekannt, dass Pyrimidin-4,6-dicarbonsäurediamide und 2,4-substituierte Pyridin-N-oxide die Enzyme Prolin- und Lysinhydroxylase inhibieren und damit eine Hemmung der Kollagenbiosynthese durch Beeinflussung der kollagenspezifischen Hydroxylierungsreaktion bewirken (EP 0418797; EP 0463592). Durch diese Hemmung der Kollagenbiosynthese wird ein nicht funktionsfähiges, unterhydroxyliertes Kollagenmolekül gebildet, das von den Zellen nur in geringer Menge in den extrazellularen Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens. Aus den Patentanmeldungen WO 02/064571 und WO 02/064080 ist bekannt, dass bestimmte Pyridin-2,4-dicarbonsäurediamide und Pyrimidin-4,6-dicarbon-säurediamide allosterische Inhibitoren von MMP 13 sein können. Gegenstand der Internationalen Patentanmeldung WO03/049738, die unter Art. 54 (3) Europäisches Patentübereinkommen fällt, sind Pyridin-2,4-dicarbonsäurediamide und Pyrimidin-4,6-dicarbonsäuredi-amide zur selektiven Inhibition der Kollagenase MMP 13.

In Erkrankungen wie Osteoarthritis und Rheuma findet eine Zerstörung des Gelenkes statt, besonders bedingt durch den proteolytischen Abbau von Kollagen durch Kollagenasen. Kollagenasen gehören zur Superfamilie der Metalloproteinasen (MP) bzw. Matrix-Metallproteinasen (MMP). MMP's spalten Kollagen, Laminin, Proteoglykane, Elastin oder Gelatin unter physiologischen Bedingungen und spielen daher eine wichtige Rolle im Knochen und Bindegewebe. Eine Vielzahl von verschiedenen Inhibitoren der MMP's, bzw. der Kollagenasen sind bekannt (EP 0 606 046; WO94/28889). Nachteile der bekannten Inhibitoren der MMP's sind häufig die mangelnde Spezifität der Hemmung für nur eine Klasse der MMP's. Daher hemmen die meisten MMP-Inhibitoren mehrere MMP's gleichzeitig, weil die katalytische Domäne der MMP's eine ähnliche Struktur aufweist. Demzufolge wirken die Inhibitoren in unerwünschter Weise auf viele Enzyme, auch solche mit vitaler Funktion ein (Massova I., et al., The FASEB Journal (1998) 12, 1075-1095).

In dem Bestreben, wirksame Verbindungen zur Behandlung von Bindegewebserkrankungen zu finden, wurde nun gefunden, dass die erfindungsgemäß eingesetzten Verbindungen starke Inhibitoren der Matrix-Metalloproteinase 13 sind, während die erfindungsgemäß eingesetzten Verbindungen im wesentlichen unwirksam sind bei den MMP's 3 und 8.

Gegenstand der Erfindung ist daher eine Verbindung der Formel I

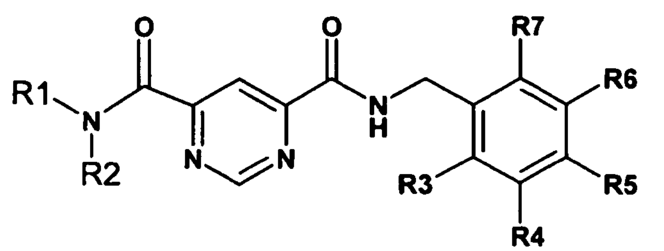

(I)

und/oder alle stereoisomeren Formen der Verbindung der Formel I und/oder Gemische dieser Formen in jedem Verhältnis, und/oder ein physiologisch verträgliches Salz der Verbindung der Formel I, wobei

R1 für Wasserstoffatom oder -($C_1$-$C_6$)-Alkyl steht,

R2 für -($C_1$-$C_6$)-Alkyl steht, wobei Alkyl ein-, zwei- oder dreifach substituiert ist durch -($C_6$-$C_{14}$)-Aryl, worin Aryl ein-, zwei- oder dreifach unabhängig voneinander substituiert ist durch

1) -($C_0$-$C_6$)-Alkyl-C(O)-N(R9)-(R10), worin R9 und R10 gleich oder verschieden sind und unabhängig voneinander

i) Wasserstoffatom oder

ii) -($C_1$-$C_6$)-Alkyl bedeuten oder

R9 und R10 zusammen mit dem Stickstoffatom, an das sie gebunden sind, einen 5-, 6- oder 7-gliedrigen gesättigten Ring bilden, wobei für ein oder zwei weitere Kohlenstoffatome auch ein Heteroatom aus der Reihe. Sauerstoff, Schwefel und Stickstoff stehen kann und im Falle von Stickstoff, die Stickstoffatome unabhängig

voneinander unsubstituiert oder durch $(C_1-C_6)$-Alkyl substituiert sein können,

2) $-(C_0-C_6)$-Alkyl-C(O)-NH-CN,
3) $-O-(C_0-C_6)$-Alkyl-C(O)-N(R9)-(R10), worin R9 und R10 die oben genannte Bedeutung haben,
4) $-(C_0-C_6)$-Alkyl-C(O)-N(R8)-$(C_0-C_6)$-Alkyl-N(R9)-(R10), worin R8

i) Wasserstoffatom
ii) $-(C_1-C_6)$-Alkyl, worin Alkyl unsubstituiert ist oder ein- zwei- oder dreifach unabhängig voneinander substituiert ist durch $-NH_2$, -CN, -OH,- C(O)-OH, -C(O)-NH-OH, $NO_2$, -C(O)-O-$(C_1-C_6)$-Alkyl oder Halogen oder
iii) -OH bedeutet, und
worin R9 und R10 die oben genannte Bedeutung haben,

5) $-(C_0-C_6)$-Alkyl-C(O)-N(R8)-$(C_0-C_6)$-Alkyl-Het, wobei R8 die oben genannte Bedeutung hat und Het für ein gesättigtes oder ungesättigtes monocyclisches oder bicyclisches, 3- bis 10-gliedriges heterocyclisches Ringsystem steht, das 1, 2 oder 3 identische oder verschiedene Ringheteroatome aus der Reihe Stickstoff, Sauerstoff und Schwefel enthält und unsubstituiert oder ein- zwei- oder dreifach unabhängig voneinander substituiert ist durch

a) Halogen,
b) Cyano,
c) Nitro,
d) Hydroxy,
e) Amino,
f) -C(O)-O-$(C_1-C_6)$-Alkyl,
g) -C(O)-OH,
h) $-(C_1-C_6)$-Alkyl, wobei Alkyl unsubstituiert oder ein-, zwei- oder dreifach substituiert ist durch Halogen,
i) $-O-(C_1-C_6)$-Alkyl, wobei Alkyl unsubstituiert oder ein-, zwei- oder dreifach substituiert ist durch Halogen oder -N(R9)-(R10),
j) =O,
k) -Het,
l) $-(C_2-C_6)$-Alkenyl, wobei Alkenyl unsubstituiert oder ein-, zwei- oder dreifach substituiert ist durch Halogen oder -N(R9)-(R10), oder
m) $-(C_2-C_6)$-Alkinyl, wobei Alkinyl unsubstituiert oder ein-, zwei- oder dreifach substituiert ist durch Halogen oder -N(R9)-(R10), oder

6) $-(C_0-C_6)$-Alkyl-C(O)-N(R8)-$(C_0-C_6)$-Alkyl-$(C_6-C_{14})$-Aryl, wobei Aryl unsubstituiert oder ein- zwei- oder dreifach unabhängig voneinander substituiert ist durch die oben genannten Reste a) bis m),

R3, R4, R5, R6 und R7 gleich oder verschieden sind und unabhängig voneinander für

1. Wasserstoffatom,
2. Halogen,
3. $-(C_1-C_6)$-Alkyl, worin Alkyl unsubstituiert oder ein-, zwei- oder dreifach durch Halogen substituiert ist,
4. $-O-(C_1-C_6)$-Alkyl, worin Alkyl unsubstituiert oder ein-, zwei- oder dreifach durch Halogen substituiert ist, oder
5. $-S-(C_1-C_6)$-Alkyl oder

R4 und R5 oder R5 und R6 zusammen mit den Kohlenstoffatomen, an die sie jeweils gebunden sind unabhängig voneinander einen 5- oder 6-gliedrigen Ring bilden, der aromatisch oder gesättigt ist und Null, ein oder zwei Heteroatome aus der Reihe Sauerstoff, Stickstoff oder Schwefel enthält, wobei der Ring unsubstituiert oder an einem oder an mehreren Kohlenstoffatomen ein- oder zweifach durch Halogen substituiert ist und die anderen Reste R3, R6,

und R7 oder R3, R4 und R7 die oben genannte Bedeutung von 1. bis 5. haben.

Ein weiterer Gegenstand der Erfindung ist eine Verbindung der Formel I, wobei

R1 für Wasserstoffatom oder -($C_1$-$C_6$)-Alkyl steht,

R2 für -($C_1$-$C_6$)-Alkyl steht, wobei Alkyl ein-, zwei- oder dreifach substituiert ist durch Phenyl, worin Phenyl ein-, zwei- oder dreifach unabhängig voneinander substituiert ist durch

1) -($C_0$-$C_6$)-Alkyl-C(O)-N(R9)-(R10), worin R9 und R10 gleich oder verschieden sind und unabhängig voneinander

i) Wasserstoffatom oder
ii) -($C_1$-$C_6$)-Alkyl bedeuten oder
R9 und R10 zusammen mit dem Stickstoffatom, an das sie gebunden sind, einen 5-, 6- oder 7-gliedrigen gesättigten Ring bilden, wobei für ein oder zwei weitere Kohlenstoffatome auch ein Heteroatom aus der Reihe Sauerstoff, Schwefel und Stickstoff stehen kann und im Falle von Stickstoff, die Stickstoffatome unabhängig voneinander unsubstituiert oder durch ($C_1$-$C_6$)-Alkyl substituiert sein können,

2) -($C_0$-$C_6$)-Alkyl-C(O)-NH-CN,
3) -O-($C_0$-$C_6$)-Alkyl-C(O)-N(R9)-(R10), worin R9 und R10 die oben genannte Bedeutung haben,
4) -($C_0$-$C_6$)-Alkyl-C(O)-N(R8)-($C_0$-$C_6$)-Alkyl-N(R9)-(R10), worin R8

i) Wasserstoffatom,
ii) -($C_1$-$C_6$)-Alkyl, worin Alkyl unsubstituiert ist oder ein- zwei- oder dreifach unabhängig voneinander substituiert ist durch -$NH_2$, -CN, -OH, -C(O)-OH, -C(O)-NH-OH, $NO_2$, -C(O)-O-($C_1$-$C_6$)-Alkyl oder Halogen oder
iii) -OH bedeutet, und

worin R9 und R10 die oben genannte Bedeutung haben,
5) -($C_0$-$C_6$)-Alkyl-C(O)-N(R8)-($C_0$-$C_6$)-Alkyl-Het, wobei R8 die oben genannte Bedeutung hat und Het für einen Rest aus der Gruppe Azepin, Azetidin, Aziridin, Benzimidazol, Benzofuran, Benzo[1,4]dioxin, 1,3-Benzodioxol, 4H-Benzo[1,4]oxazin, Benzoxazol, Benzothiazol, Benzothiophen, Chinazolin, Chinolin, Chinoxalin, Chroman, Cinnolin, 1,2-Diazepin, 1,3-Diazepin, 1,4-Diazepin, 1,4-Dioxin, Dioxol, Furan, Imidazol, Indazol, Indol, Isochinolin, Isochroman, Isoindol, Isothiazol, Isoxazol, Morpholin, 1,2-Oxazin, 1,3-Oxazin, 1,4-Oxazin, Oxazol, Oxiran, Piperazin, Piperidin, Phthalazin, Pyran, Pyrazin, Pyrazol, Pyridazin, Pyridin, Pyrimidin, Pyridoimidazol, Pyridopyridin, Pyridopyrimidin, Pyrrol, Pyrrolidin, Tetrazol, 1,2-Thiazin, 1,3-Thiazin, 1,4-Thiazin, Thiazol, Thiomorpholin, Thiophen, Thiopyran, 1,2,3-Triazin, 1,3,5-Triazin, 1,2,4-Triazin. 1,2,3-Triazol oder 1,2,4-Triazol steht und worin Het unsubstituiert ist oder ein- zwei- oder dreifach unabhängig voneinander substituiert ist durch

a) Halogen,
b) Cyano,
c) Nitro,
d) Hydroxy,
e) Amino,
f) -C(O)-O-($C_1$-$C_6$)-Alkyl,
g) -C(O)-OH,
h) -($C_1$-$C_6$)-Alkyl, wobei Alkyl unsubstituiert oder ein-, zwei- oder dreifach substituiert ist durch Halogen,
i) -O-($C_1$-$C_6$)-Alkyl, wobei Alkyl unsubstituiert oder ein-, zwei- oder dreifach substituiert ist durch Halogen oder -N(R9)-(R10),
j) =O,
k) -Het, worin Het wie oben definiert ist,
l) -($C_2$-$C_6$)-Alkenyl, wobei Alkenyl unsubstituiert oder ein-, zwei- oder dreifach

substituiert ist durch Halogen oder -N(R9)-(R10), oder
m) -($C_2$-$C_6$)-Alkinyl, wobei Alkinyl unsubstituiert oder ein-, zwei- oder dreifach substituiert ist durch Halogen oder -N(R9)-(R10), oder

6) -($C_0$-$C_6$)-Alkyl-C(O)-N(R8)-($C_0$-$C_6$)-Alkyl-($C_6$-$C_{14}$)-Phenyl, wobei Phenyl unsubstituiert oder ein- zwei- oder dreifach unabhängig voneinander substituiert ist durch die oben genannten Reste a) bis m),

R3, R4, R5, R6 und R7 gleich oder verschieden sind und unabhängig voneinander für

1. Wasserstoffatom,
2. Halogen,
3. -($C_1$-$C_6$)-Alkyl, worin Alkyl unsubstituiert oder ein-, zwei- oder dreifach durch Halogen substituiert ist, oder
4. -O-($C_1$-$C_6$)-Alkyl, worin Alkyl unsubstituiert oder ein-, zwei- oder dreifach durch Halogen substituiert ist, stehen, oder

R4 und R5 oder R5 und R6 zusammen mit den Kohlenstoffatomen, an die sie jeweils gebunden sind unabhängig voneinander einen Dioxan-, Dioxol-, Dihydrofuran- oder Furan-Ring bilden, wobei der Ring unsubstituiert oder an einem oder an mehreren Kohlenstoffatomen ein- oder zweifach durch Halogen substituiert ist und die anderen Reste R3, R6, und R7 oder R3, R4 und R7 die oben genannte Bedeutung von 1. bis 4. haben.

Ein weiterer Gegenstand der Erfindung ist eine Verbindung der Formel I, wobei

R1 für Wasserstoffatom steht,
R2 für -($C_1$-$C_3$)-Alkyl steht, wobei Alkyl substituiert ist durch Phenyl, worin Phenyl ein-, zwei- oder dreifach unabhängig voneinander substituiert ist durch

1) -($C_0$-$C_6$)-Alkyl-C(O)-N(R9)-(R10), worin R9 und R10 für Wasserstoffatom, Methyl, Ethyl, Propyl oder Butyl steht, oder
R9 und R10 zusammen mit dem Stickstoffatom, an das sie gebunden sind einen Rest bilden, der sich von Pyrrolidin, Piperidin, Pyrazolidin, Pyrazin, Tetrazin, Imidazolidin, Piperazin, Isoxazolidin, Morpholin, Isothiazolidin oder Thiomorpholin ableiten lässt und im Falle von Stickstoff, die Stickstoffatome unabhängig voneinander unsubstituiert oder durch ($C_1$-$C_4$)-Alkyl substituiert sein können,
2) -($C_0$-$C_4$)-Alkyl-C(O)-NH-CN,
3) -O-($C_0$-$C_6$)-Alkyl-C(O)-N(R9)-(R10), worin R9 und R10 die oben genannte Bedeutung haben,
4) -($C_0$-$C_6$)-Alkyl-C(O)-N(R8)-($C_0$-$C_6$)-Alkyl-N(R9)-(R10), worin R8, für Wasserstoff, Methyl, Ethyl, Propyl oder Butyl steht, und R9 und R10 die oben genannte Bedeutung haben,
5) -C(O)-N(R8)-($C_0$-$C_2$)-Alkyl-Het, wobei R8 die oben genannte Bedeutung hat und Het für Azepin, Azetidin, Aziridin, Benzimidazol, Benzofuran, Benzo[1,4]dioxin, 1,3-Benzodioxol, 4H-Benzo[1,4]oxazin, Benzoxazol, Benzothiazol, Benzothiophen, Chinazolin, Chinolin, Chinoxalin, Chroman, Cinnolin, 1,2-Diazepin, 1,3-Diazepin, 1,4-Diazepin, 1,4-Dioxin, Dioxol, Furan, Imidazol, Indazol, Indol, Isochinolin, Isochroman, Isoindol, Isothiazol, Isoxazol, Morpholin, 1,2-Oxazin, 1,3-Oxazin, 1,4-Oxazin, Oxazol, Oxiran, Piperazin, Piperidin, Phthalazin, Pyran, Pyrazin, Pyrazol, Pyridazin, Pyridin, Pyrimidin, Pyridoimidazol, Pyridopyridin, Pyridopyrimidin, Pyrrol, Pyrrolidin, Tetrazol, 1,2-Thiazin, 1,3-Thiazin, 1,4-Thiazin, Thiazol, Thiomorpholin, Thiophen, Thiopyran, 1,2,3-Triazin, 1,3,5-Triazin, 1,2,4-Triazin, 1,2,3-Triazol oder 1,2,4-Triazol steht und Het unsubstituiert oder ein- zwei- oder dreifach unabhängig voneinander substituiert ist durch

a) Halogen,
b) Cyano,
c) Nitro,
d) Hydroxy,
e) Amino,

f) -C(O)-O-($C_1$-$C_4$)-Alkyl,
g) -C(O)-OH,
h) -($C_1$-$C_4$)-Alkyl, wobei Alkyl unsubstituiert oder ein-, zwei- oder dreifach substituiert ist durch Halogen,
i) -O-($C_1$-$C_4$)-Alkyl, wobei Alkyl unsubstituiert oder ein-, zwei- oder dreifach substituiert ist durch Halogen, oder

6) -C(O)-N(R8)-($C_0$-$C_4$)-Alkyl-Phenyl, wobei Phenyl unsubstituiert oder ein- zwei- oder dreifach unabhängig voneinander substituiert ist durch die oben genannten Reste a) bis i),

R3, R4, R5, R6 und R7 gleich oder verschieden sind und unabhängig voneinander für

1. Wasserstoffatom,
2. Halogen,
3. -($C_1$-$C_6$)-Alkyl, worin Alkyl unsubstituiert oder ein-, zwei- oder dreifach durch Halogen substituiert ist,
4. -O-($C_1$-$C_6$)-Alkyl, stehen, worin Alkyl unsubstituiert oder ein-, zwei- oder dreifach durch Halogen substituiert ist, oder

R4 und R5 oder R5 und R6 zusammen mit den Kohlenstoffatomen, an die sie gebunden sind unabhängig voneinander einen Dioxan-, Dioxol-, Dihydrofuran- oder Furan-Ring bilden und die anderen Reste R3, R6, und R7 oder R3, R4 und R7 die oben genannte Bedeutung von 1. bis 4. haben.

Ein weiterer Gegenstand der Erfindung ist eine Verbindung der Formel I, wobei eine der folgenden Verbindungen ausgewählt wird.

Pyrimidin-4,6-dicarbonsäure 4-(4-diethylcarbamoyl-benzylamid) 6-(3-methoxy-benzylamid),
Pyrimidin-4,6-dicarbonsäure 4-(3-methoxy-benzylamid) 6-(4-propylcarbamoyl-benzylamid),
Pyrimidin-4,6-dicarbonsäure 4-(4-isopropylcarbamoyl-benzylamid) 6-(3-methoxybenzylamid),
Pyrimidin-4,6-dicarbonsäure 4-[4-(2-dimethylamino-ethylcarbamoyl)-benzylamid] 6-(3-methoxy-benzylamid),
Pyrimidin-4,6-dicarbonsäure 4-[(2,3-dihydro-benz[1,4]dioxin-6-ylmethyl)-amid] 6-[4-(2-dimethylamino-ethylcarbamoyl)-benzylamid],
Pyrimidin-4,6-dicarbonsäure 4-(3-chlor-4-fluor-benzylamid) 6-[4-(2-dimethylamino-ethylcarbamoyl)-benzylamid],
Pyrimidin-4,6-dicarbonsäure 4-(4-fluor-3-methyl-benzylamid) 6-{4-[2-(4-methyl-piperazin-1-yl)-2-oxo-ethyl]-benzylamid},
Pyrimidin-4,6-dicarbonsäure 4-(4-fluor-3-methyl-benzylamid) 6-[4-(2-morpholin-4-yl-2-oxo-ethoxy)-benzylamid],
Pyrimidin-4,6-dicarbonsäure 4-(4-diethylcarbamoylmethoxy-benzylamid) 6-(4-fluor-3-methyl-benzylamid),
Pyrimidin-4,6-dicarbonsäure 4-(4-fluor-3-methyl-benzylamid) 6-[4-(isopropylcarbamoylmethyl)-benzylamid],
Pyrimidin-4,6-dicarbonsäure 4-(4-fluor-3-methyl-benzylamid)6-{4-[(2-morpholin-4-yl-ethylcarbamoyl)-methyl]-benzylamid},
Pyrimidin-4,6-dicarbonsäure 4-(4-diethylcarbamoylmethyl-benzylamid)6-(4-fluor-3-methyl-benzylamid),
Pyrimidin-4,6-dicarbonsäure 4-(4-fluor-3-methyl-benzylamid) 6-[4-(2-morpholin-4-yl-2-oxo-ethyl)-benzylamid],
Pyrimidin-4,6-dicarbonsäure 4-(4-fluor-3-methyl-benzylamid) 6-[4-(isopropylcarbamoylmethoxy)-benzylamid],
Pyrimidin-4,6-dicarbonsäure 4-(3-methoxy-benzylamid)6-[4-(2-morpholin-4-yl-ethylcarbamoyl)-benzylamid],
Pyrimidin-4,6-dicarbonsäure 4-(3-methoxy-benzylamid)6-[4-(2-pyrrolidin-1-yl-ethylcarbamoyl)-benzylamid],
Pyrimidin-4,6-dicarbonsäure 4-(4-fluor-3-methyl-benzylamid) 6-[4-(thiomorpholin-4-carbonyl)-benzylamid],
Pyrimidin-4,6-dicarbonsäure 4-(3-methoxy-benzylamid) 6-[4-(thiomorpholin-4-carbonyl)-benzylamid],
Pyrimidin-4,6-dicarbonsäure 4-(4-cyancarbamoyl-benzylamid)6-(4-fluor-3-methyl-benzylamid),
Pyrimidin-4,6-dicarbonsäure 4-(3-methoxy-benzylamid) 6-[4-(3-morpholin-4-yl-propylcarbamoyl)-benzylamid],
Pyrimidin-4,6-dicarbonsäure 4-[(2,3-dihydro-benzofuran-5-ylmethyl)-amid]6-[4-(3-morpholin-4-yl-propylcarbamoyl)-benzylamid],
Pyrimidin-4,6-dicarbonsäure 4-(4-fluor-3-methyl-benzylamid) 6-[4-(4-methyl-piperazin-1-carbonyl)-benzylamid],
Pyrimidin-4,6-dicarbonsäure 4-[(2,3-dihydro-benzofuran-5-ylmethyl)-amid] 6-{4-[(pyridin-4-ylmethyl)-carbamoyl]-benzylamid},
Pyrimidin-4,6-dicarbonsäure 4-(4-N-cyan-carbamoyl-benzylamid)6-[(2,3-dihydro-benzfuran-5-ylmethyl)-amid],
Pyrimidin-4,6-dicarbonsäure 4-(4-N-cyan-carbamoyl-benzylamid)6-(3-methoxy-benzylamid),
Pyrimidin-4,6-dicarbonsäure 4-(4-fluor-3-methyl-benzylamid) 6-[4-(morpholin-4-carbonyl)-benzylamid],

Pyrimidin-4,6-dicarbonsäure 4-(4-fluor-3-methyl-benzylamid) 6-[4-(2-piperazin-1-yl-ethylcarbamoyl)-benzylamid],
Pyrimidin-4,6-dicarbonsäure 4-(4-tert-butylcarbamoyl-benzylamid)6-(3-methoxy-benzylamid),
Pyrimidin-4,6-dicarbonsäure 4-(3-methoxy-benzylamid)6-{4-[methyl-(1-methyl-piperidin-4-yl)-carbamoyl]-benzylamid},
Pyrimidin-4,6-dicarbonsäure 4-(4-fluor-3-methyl-benzylamid)6-[4-(2-pyrrolidin-1-yl-ethylcarbamoyl)-benzylamid],
Pyrimidin-4,6-dicarbonsäure 4-(3-methoxy-benzylamid) 6-[3-(2-morpholin-4-yl-ethylcarbamoyl)-benzylamid],
Pyrimidin-4,6-dicarbonsäure 4-(4-fluor-3-methyl-benzylamid) 6-[4-(2-morpholin-4-yl-ethylcarbamoyl)-benzylamid],
[4-({[6-(4-Fluor-3-methyl-benzylcarbamoyl)-pyrimidin-4-carbonyl]-amino}-methyl)-benzoylamino]-acetic acid methyl ester,
Pyrimidin-4,6-dicarbonsäure 4-(3-methoxy-benzylamid) 6-[3-(morpholin-4-carbonyl)-benzylamid],
Pyrimidin-4,6-dicarbonsäure 4-(3-methoxy-benzylamid) 6-{4-[(piperidin-4-ylmethyl)-carbamoyl]-benzylamid},
Pyrimidin-4,6-dicarbonsäure 4-(3-methoxy-benzylamid) 6-[4-(piperidin-4-ylcarbamoyl)-benzylamid],
Pyrimidin-4,6-dicarbonsäure 4-(4-fluor-3-methyl-benzylamid) 6-[4-(piperidin-4-ylcarbamoyl)-benzylamid],
Pyrimidin-4,6-dicarbonsäure 4-(4-fluor-3-methyl-benzylamid) 6-{4-[methyl-(1-methylpiperidin-4-yl)-carbamoyl]-benzylamid},
Pyrimidin-4,6-dicarbonsäure 4-(4-fluor-3-methyl-benzylamid)6-{4-[(piperidin-4-ylmethyl)-carbamoyl]-benzylamid},
Pyrimidin-4,6-dicarbonsäure4-(3-methoxy-benzylamid)6-[4-(4-methyl-piperazin-1-carbonyl)-benzylamid],
Pyrimidin-4,6-dicarbonsäure 4-[(2,3-dihydro-benzofuran-5-ylmethyl)-amid]6-[4-(morpholin-4-carbonyl)-benzylamid],
Pyrimidin-4,6-dicarbonsäure 4-[(2,3-dihydro-benzofuran-5-ylmethyl)-amid]6-[4-(4-methylpiperazin-1-carbonyl)-benzylamid],
Pyrimidin-4,6-dicarbonsäure 4-[(2,3-dihydro-benzfuran-5-ylmethyl)-amid]6-[4-(2-pyrrolidin-1-yl-ethylcarbamoyl)-benzylamid],
Pyrimidin-4,6-dicarbonsäure 4-[(2,3-dihydro-benzofuran-5-ylmethyl)-amid]6-[4-(2-morpholin-4-yl-ethylcarbamoyl)-benzylamid],
Pyrimidin-4,6-dicarbonsäure 4-(3-chlor-4-fluor-benzylamid)6-[4-(2-pyrrolidin-1-yl-ethylcarbamoyl)-benzylamid],
Pyrimidin-4,6-dicarbonsäure 4-(3-methoxy-benzylamid)6-[4-(morpholin-4-carbonyl)-benzylamid],
Pyrimidin-4,6-dicarbonsäure 4-(3-methoxy-benzylamid)6-{4-[(pyridin-4-ylmethyl)-carbamoyl]-benzylamid},
Pyrimidin-4,6-dicarbonsäure 4-(3-chlor-4-fluor-benzylamid)6-(4-diethylcarbamoyl-benzylamid),
Pyrimidin-4,6-dicarbonsäure 4-(3-chlor-4-fluor-benzylamid)6-[4-(morpholin-4-carbonyl)-benzylamid],
Pyrimidin-4,6-dicarbonsäure 4-(3-chlor-4-fluor-benzylamid)6-[4-(2-morpholin-4-yl-ethylcarbamoyl)-benzylamid] oder
Pyrimidin-4,6-dicarbonsäure 4-(4-diethylcarbamoyl-benzylamid)6-(3-methoxy-benzylamid).

Unter dem Begriff "Halogen" wird Fluor, Chlor, Brom oder Jod verstanden.
Unter dem Begriff "-($C_0$-$C_6$)-Alkyl" werden Kohlenwasserstoffreste verstanden, deren Kohlenstoffkette geradkettig oder verzweigt ist und 1 bis 6 Kohlenstoffatome enthält, beispielsweise Methyl, Ethyl, Propyl, i-Propyl, Butyl, tertiär-Butyl, Pentyl oder Hexyl. "-$C_0$-Alkyl" ist eine kovalente Bindung.
Unter dem Begriff "-($C_6$-$C_{14}$)-Aryl" werden aromatische Kohlenstoffreste verstanden mit 6 bis 14 Kohlenstoffatomen im Ring. -($C_6$-$C_{14}$)-Arylreste sind beispielsweise Phenyl, Naphthyl, zum Beispiel 1-Naphthyl, 2-Naphthyl, Biphenylyl, zum Beispiel 2-Biphenylyl, 3-Biphenylyl und 4-Biphenylyl, Anthryl oder Fluorenyl. Biphenylylreste, Naphthylreste und insbesondere Phenylreste sind bevorzugte Arylreste.
Unter dem Begriff "-R4 und R5 oder R5 und R6 zusammen mit den Kohlenstoffatomen, an die sie jeweils gebunden sind unabhängig voneinander einen 5- oder 6-gliedrigen Ring bilden, der aromatisch oder gesättigt ist und Null, ein oder zwei Heteroatome aus der Reihe Sauerstoff, Stickstoff oder Schwefel enthält" werden Ringsysteme verstanden die sich von Dioxol, Pyrrol, Pyrrolidin, Pyridin, Piperidin, Dioxan, Tetrahydropyridin, Pyrazol, Imidazol, Pyrazolin, Imidazolin, Pyrazolidin, Imidazolidin, Pyridazin, Pyrimidin, Pyrazin, Piperazin, Pyran, Furan, Dihydrofuran, Tetrahydrofuran, Oxazol, Isoxazol, 2-Isoxazolin, Isoxazolidin, Morpholin, Oxothiolan, Thiopyran, Thiazol, Isothiazol, 2-Isothiazolin, Isothiazolidin oder Thiomorpholin ableiten lassen.
Unter dem Begriff "Het" wird ein gesättigtes oder ungesättigtes monocyclisches oder bicyclisches, 3- bis 10-gliedriges heterocyclisches Ringsystem verstanden, das 1, 2 oder 3 identische oder verschiedene Ringheteroatome aus der Reihe Stickstoff, Sauerstoff und Schwefel enthält. Het enthält im zugrunde liegenden monocyclischen oder bicyclischen heterocyclischen Ringsystem 3, 4, 5, 6, 7, 8, 9 oder 10 Ringatome. Das monocyclische Ringsystem kann ein 3-, 4-, 5-, 6- oder 7-gliedriger Ring sein. Im bicyclischen Het können zwei Ringe miteinander verknüpft sein, von denen einer ein 5-gliedriger oder 6-gliedriger heterocyclischer Ring sein kann und der andere ein 5- oder 6-gliedriger heterocyclischer oder carbocyclischer Ring sein kann. Eine bicyclische Het Gruppe kann beispielsweise aus 8, 9 oder 10 Ringatomen bestehen.
Het beinhaltet gesättigte heterocyclische Ringsysteme, die in den Ringen keine Doppelbindung besitzen und ebenso

ungesättigte heterocyclische Ringsysteme einschließlich monoungesättige und poly-ungesättigte heterocyclische Ringsysteme, die eine oder mehrere Doppelbindungen besitzen und ein stabiles Ringsystem ausbilden. Ungesättigte Ringe können teilweise ungesättigt sein oder ein aromatisches System bilden. In der Het Gruppe sind identische oder verschiedene Heteroatome aus der Reihe Stickstoff, Sauerstoff und Schwefel. Beispiele von Heterocyclen, aus denen die Het Gruppe abgeleitet werden kann sind Acridinyl, Azocinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1 2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Chinazolinyl, Chinolinyl, 4H-Chinolizinyl, Chinoxalinyl, Chinuclidinyl, Tetrahydrofuranyl, Tetrahydroisochinolinyl, Tetrahydrochinolinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl und Xanthenyl.
Bevorzugt sind Azepin, Azetidin, Aziridin, Benzimidazol, Benzofuran, Benzo[1,4]dioxin, 1,3-Benzodioxol, 4H-Benzo[1,4]oxazin, Benzoxazol, Benzothiazol, Benzothiophen, Chinazolin, Chinolin, Chinoxalin, Chroman, Cinnolin, 1,2-Diazepin, 1,3-Diazepin, 1,4-Diazepin, 1,4-Dioxin, Dioxol, Furan, Imidazol, Indazol, Indol, Isochinolin, Isochroman, Isoindol, Isothiazol, Isoxazol, Morpholin, 1,2-Oxazin, 1,3-Oxazin, 1,4-Oxazin, Oxazol, Oxiran, Piperazin, Piperidin, Phthalazin, Pyran, Pyrazin, Pyrazol, Pyridazin, Pyridin, Pyrimidin, Pyridoimidazol, Pyridopyridin, Pyridopyrimidin, Pyrrol, Pyrrolidin, Tetrazol, 1,2-Thiazin, 1,3-Thiazin, 1,4-Thiazin, Thiazol, Thiomorpholin, Thiophen, Thiopyran, 1,2,3-Triazin, 1,3,5-Triazin, 1,2,4-Triazin, 1,2,3-Triazol oder 1,2,4-Triazol etc. und ebenso Ringsysteme, die sich aus den aufgeführten Heterocyclen durch Verknüpfung oder Ankondensation mit einem carbocyclischen Ring ergeben, wie beispielsweise benzo-anellierte, cyclopenta-kondensierte, cyclohexa-kondensierte or cyclohepta-kondensierte Derivate dieser Heterocyclen. Geeignete Stickstoffheterocyclen können ebenfalls als N-Oxide oder als quartäre Salze, in denen ein geeignetes Stickstoffatom durch ($C_1$-$C_4$)-Alkylreste alkyliert ist, vorliegen.
Die Het Gruppen können unsubstituiert oder entsprechend den aufgeführten Definitionen substituiert sein.

Unter dem Begriff "R9 und R10 zusammen mit dem Stickstoffatom, an das sie gebunden sind, einen 5-, 6- oder 7-gliedrigen gesättigten Ring bilden, wobei für ein oder zwei weitere Kohlenstoffatome auch ein Heteroatom aus der Reihe Sauerstoff, Schwefel und Stickstoff stehen kann" werden Reste verstanden, die sich von Imidazolidin, Isothiazolidin, Isoxazolidin, Morpholin, Piperazin, Piperidin, Pyrazin, Pyrazolidin, Pyrrolidin, Tetrazin, oder Thiomorpholin ableiten lassen.

Die Verbindungen der Formel I lassen sich beispielsweise dadurch herstellen, dass man eine Verbindung der Formel II

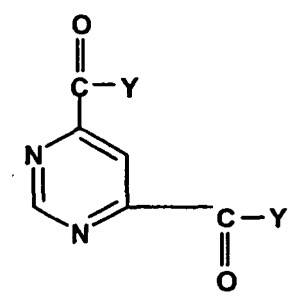

(II)

a) mit einer Verbindung der Formel IIIa oder IIIb

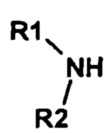

IIIa

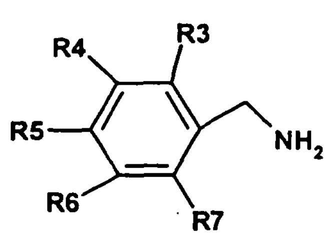

IIIb

umsetzt, wobei R1, R2, R3, R4, R5, R6 und R7 die in Formel I angegebenen Bedeutungen haben und Y Halogen, Hydroxyl oder $C_1$-$C_4$-Alkoxy ist oder zusammen mit der Carbonylgruppe einen Aktivester oder ein gemischtes Anhydrid bildet, wobei eine Verbindung der Formel I gebildet wird und die Reaktionsprodukte gegebenenfalls in ihre physiologisch verträglichen Salze überführt, oder

b) eine Verbindung der Formel II mit einer Verbindung der Formel IIIa oder IIIb zu einer Verbindung der Formel IVa oder IVb.

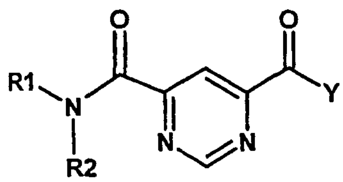

(IVa)

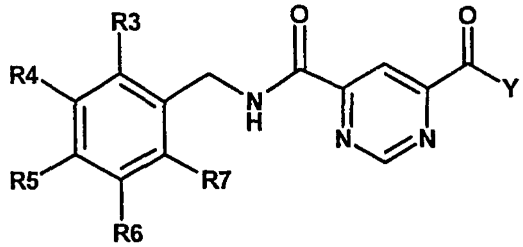

(IVb)

umsetzt, wobei R1 bis R7 die in Formel I angegebenen Bedeutungen haben und Y Halogen, Hydroxyl oder $C_1$-$C_4$-Alkoxy ist oder zusammen mit der Carbonylgruppe einen Aktivester oder ein gemischtes Anhydrid bildet und die Verbindung der Formel IVa oder IVb gegebenenfalls reinigt und anschließend mit einer Verbindung der Formel IIIa oder IIIb in eine Verbindung der Formel I überführt.

Im Folgenden wird die Herstellung von Verbindungen gemäß Formel I und die Herstellung der dafür benötigten Ausgangssubstanzen-sofern sie nicht käuflich sind - näher beschrieben.

Die Herstellung der erfindungsgemäßen Verbindungen gelingt am einfachsten dadurch, dass die beiden Komponenten, das Pyrimidin-Derivat gemäß Formel (II) und das Amin gemäß Formel IIIa oder IIIb in äquimolaren Mengen zusammengegeben werden und bei Temperaturen zwischen -30 °C bis 150 °C, bevorzugt bei 20 °C bis 100 °C zu Verbindung der Formel IVa oder IVb umgesetzt werden und anschließend die Verbindungen der Formel IVa oder IVb mit bis zu einer äquimolaren Menge vom Amin gemäß Formel IIIb oder IIIa in analoger Weise umgesetzt werden. Die Beendigung der Reaktion lässt sich beispielsweise mittels Dünnschichtchromatographie oder HPLC-MS bestimmen. Eine Variante dieses Verfahrens besteht darin, dass man in einem geeigneten Lösungsmittel, wie Diethylether, Dimethoxyethan oder Tetrahydrofuran, chlorierten Kohlenwasserstoffen wie Methylenchlorid, Chloroform, Tri- oder Tetrachlorethylen, Benzol, Toluol oder auch polaren Lösungsmitteln wie Dimethylformamid, Aceton oder Dimethylsulfoxid arbeitet. Die Reaktionstemperaturen liegen dabei zwischen Raumtemperatur und dem Siedepunkt des Lösungsmittels, wobei Temperaturen im Bereich von Raumtemperatur bis 130 °C besonders bevorzugt sind.

Ebenso kann die Umsetzung über ein gemischtes Anhydrid wie Chlorameisensäure-ethylester oder über einen aktiven Ester wie Paranitrophenylester (Y = $ClCH_2$-COO oder $NO_2$-$C_6H_4$-O) erfolgen. Entsprechende Methoden sind bekannt und in der Literatur beschrieben.

Ebenso kann die Umsetzung einer Verbindung der Formel II oder einer Verbindung der Formel IVa oder IVb mit einem Amin der Formel IIIa oder IIIb erfolgen, falls Y für OH steht und die entsprechende Carbonsäure durch gebräuchliche Kopplungsreagenzien in situ aktiviert wird. Solche Kopplungsreagentien sind beispielsweise Carbodiimide wie Dicyclohexylcarbodiimid (DCC) oder Diisopropylcarbodiimid (DCI) oder N,N'-Carbonyldiazole wie N,N'-Carbonyldiimidazol oder ein Uronium Salz wie 0-((Cyano(ethoxycarbonyl)methylen)-amino)-1,1,3,3-tetramethyluronium tetrafluoroborat (TOTU) oder 0-(7-Azabenzotriazol-1-yl)-1,1,3,3-tetramethyluronium hexafluorophosphat (HATU). Entsprechende Methoden sind bekannt. Falls Amine der Formel IIIa oder IIIb nicht käuflich sind, lassen sie sich aus entsprechenden käuflichen Ausgangsverbindungen nach literaturbekannten Methoden herstellen. Geeignete Ausgangsverbindungen für Amine sind beispielsweise Nitrile, Nitro-Verbindungen, Carbonsäureamide, Carbonsäureester, Carbonsäuren, Aldehyde und Bromide. Nitrile, Nitro-Verbindungen und Carbonsäureamide können nach bekannten Methoden zu Aminen reduziert werden. Carbonsäuren und Carbonsäureester können in die Carbonsäureamide überführt werden. Aldehyde können über eine reduktive Aminierung mit $NH_4Ac/NaBH_4$ direkt zu den Aminen überführt werden oder mit Hydroxylamin zunächst zu den Oximen und dann durch Reduktion zu den Aminen überführt werden.

Gegebenenfalls kann die Umsetzung auch in Gegenwart von Basen erfolgen. Als zusätzliche Basen kommen beispielsweise Carbonate oder Hydrogencarbonate wie Natrium- oder Kaliumcarbonat oder Natrium- oder Kaliumhydrogencarbonat, oder tertiäre Amine, wie Triethylamin, Tributylamin, Ethyldiisopropylamin oder heterocyclische Amine

wie N-Alkylmorpholin, Pyridin, Chinolin oder Dialkylaniline in Betracht.

Gegebenenfalls kann die Aufarbeitung der Produkte, insbesondere die Verbindung der Formel IVa oder IVb, beispielsweise durch Extraktion oder Chromatographie z. B. über Kieselgel erfolgen. Das isolierte Produkt kann umkristallisiert und gegebenenfalls mit einer geeigneten Säure zu einem physiologisch verträglichen Salz umgesetzt werden. Als geeignete Säuren kommen beispielsweise in Betracht:

Mineralsäuren, wie Chlorwasserstoff- und Bromwasserstoffsäure sowie Schwefel-, Phosphor-, Salpeter- oder Perchlorsäure oder organische Säuren wie Ameisen-, Essig-, Propion-, Bernstein-, Glykol-, Milch-, Äpfel-, Wein-, Zitronen-, Malein-, Fumar-, Phenylessig-, Benzoe-, Methansulfon-, Toluolsulfon-, Oxal-, 4-Aminobenzoe-, Naphthalin-1,5-disulfon- oder Ascorbinsäure.

Die Ausgangsverbindungen der Formel IIIa oder IIIb können, soweit sie nicht käuflich sind, einfach synthetisiert werden (z. B. Organikum, Organisch Chemisches Grundpraktikum, 15. Aufl., VEB Deutscher Verlag der Wissenschaften, 1976; eine Übersicht über die verschiedenen Möglichkeiten findet sich im Methodenregister, S. 822).

Die Ausgangsverbindungen der Formel (II) erhält man beispielsweise durch Umsetzung von Pyrimidin-4,6-dicarbonsäure, zu dem entsprechenden Pyrimidin-4,6-dicarbonsäurehalogenid, bevorzugt-chlorid (nach literaturbekannten Verfahren), vorzugsweise in Gegenwart eines Katalysators wie Dimethylformamid. Dieses Säurehalogenid kann dann beispielsweise entweder mit einem geeigneten Alkohol, z. B. Paranitrobenzylalkohol zu dem entsprechenden Aktivester, oder aber mit niederen Alkoholen wie Methanol oder Ethanol zu den entsprechenden Estern umgesetzt werden. Ebenso kann die Pyrimidin-4,6-dicarbonsäure auch zunächst unter Zusatz einer geeigneten Carbonsäure oder eines Carbonsäureesters wie Chlorameisensäureethylester in ein gemischtes Anhydrid überführt werden, welches dann mit den Aminen der Verbindung der Formeln IIIa oder IIIb und (IVa oder IVb zu den erfindungsgemäßen Produkten umgesetzt wird. Eine entsprechende Methode ist ebenfalls in der Literatur beschrieben.

Die Herstellung der Pyrimidin-4,6-dicarbonsäure erfolgt nach literaturbekannten Verfahren, beispielsweise durch Oxidation von 4,6-Dimethylpyrimidin, welches seinerseits beispielsweise erhältlich ist durch katalytische Hydrierung von käuflich erhältlichem 2-Mercapto-4,6-dimethylpyrimidin.

Sofern Verbindungen der Formel I diastereoisomere oder enantiomere Formen zulassen und bei der gewählten Synthese als deren Gemische anfallen, gelingt die Trennung in die reinen Stereoisomeren entweder durch Chromatographie an einem gegebenenfalls chiralen Trägermaterial, oder, sofern die racemische Verbindung der Formel I zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L- und D-Arginin die unterschiedlich löslichen diastereomeren Salze gebildet, die schwerer lösliche Komponente als Feststoff isoliert, das leichter lösliche Diastereomer aus der Mutterlauge abgeschieden, und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D- und L-Milchsäure sowie (+) und (-)-Mandelsäure in die reinen Enantiomeren überführen. Auch kann man chirale Verbindungen, die Alkohol- oder Aminfunktionen enthalten, mit entsprechend aktivierten oder gegebenenfalls N- geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der isomeren genutzt werden, indem man eine Trennung der nunmehr vorliegenden Diastereomeren durch Kristallisation oder Chromatographie an geeigneten Stationärphasen vornimmt und danach den mitgeführten chiralen Molekülteil mittels geeigneter Methoden wieder abspaltet.

Saure oder basische Produkte der Verbindung der Formel I können in Form ihrer Salze oder in freier Form vorliegen. Bevorzugt sind pharmakologisch verträgliche Salze, z. B. Alkali- oder Erdalkalimetallsalze bzw. Hydrochloride, Hydrobromide, Sulfate, Hemisulfate, alle möglichen Phosphate sowie Salze der Aminosäuren, natürlicher Basen oder Carbonsäuren.

Die Herstellung physiologisch verträglicher Salze aus zur Salzbildung befähigten Verbindungen der Formel I, einschließlich deren stereoisomeren Formen, erfolgt in an sich bekannter Weise. Die Carbonsäuren bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Ornithin oder Arginin, stabile Alkali-, Erdalkali oder gegebenenfalls substituierte Ammoniumsalze. Sofern die

Verbindungen der Formel I basische Gruppen aufweist, lassen sich mit starken Säuren auch stabile Säureadditionssalze herstellen. Hierfür kommen sowohl anorganische als auch organische Säuren, wie Chlorwasserstoff-, Bromwasserstoff-, Schwefel-, Phosphor-, Methansulfon-, Benzolsulfon-, p-Toluolsulfon-, 4-Brombenzol-sulfon-, Cyclohexylamidosulfon-, Trifluormethylsulfon-, Essig-, Oxal-, Wein-, Bernstein- oder Trifluoressigsäure in Frage.

Aufgrund der pharmakologischen Eigenschaften eignen sich die Verbindungen der Formel I zur Prophylaxe und Therapie all solcher Erkrankungen, an deren Verlauf eine verstärkte Aktivität von Matrix-Metalloproteinase 13 beteiligt ist.

Dazu gehören degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Ferner gehören dazu auch Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels oder Krebserkrankungen wie Brustkrebs.

Die Applikation der erfindungsgemäßen Arzneimittel kann durch subkutane, intraartikuläre, intraperitoneale oder intravenöse Injektion erfolgen. Bevorzugt ist die intraartikuläre Injektion. Die rektale, orale, inhalative oder transdermale Applikation ist auch möglich.

Die Verbindungen der Formel I werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wässrige alkoholische oder ölige Suspensionen oder wässrige oder ölige Lösungen. Als inerte Trägerstoffe können z. B. Gummi arabicum, Magnesia, Magnesiumcarbonat, Kaliumphosphat, Milchzucker, Glukose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen, intraartikulären, intraperitonealen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z. B. in Frage physiologische Kochsalzlösung oder Alkohole, z. B. Ethanol, Propanol, Glycerin, daneben auch Zuckerlösungen wie Glukose- oder Mannitlösungen, oder auch eine Mischung aus den verschiedenen genannten Lösungsmitteln.

Ferner finden übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Die Verbindungen der Formel I werden bevorzugt als pharmazeutische Präparate in Dosierungseinheiten hergestellt und verabreicht, wobei jede Einheit als aktiven Bestandteil eine bestimmte Dosis der Verbindung der Formel I enthält. Sie können zu diesem Zweck oral in Dosen von 0,01 mg/kg/Tag bis 25,0 mg/kg/Tag, vorzugsweise 0,01 mg/kg/Tag bis 5,0 mg/kg/Tag oder parenteral in Dosen von 0,001 mg/kg/Tag bis 5 mg/kg/Tag, vorzugsweise 0,001 mg/kg/Tag bis 2,5 mg/kg/Tag, appliziert werden. Die Dosierung kann in schweren Fällen auch erhöht werden. In vielen Fällen genügen jedoch auch geringere Dosen. Diese Angaben beziehen sich auf die Behandlung eines Erwachsenen.

Nachfolgend ist die Erfindung an Hand von Beispielen näher erläutert.

**Referenz Beispiel 1:**

[4-({[6-(4-Fluoro-3-methyl-benzylcarbamoyl)-pyrimidin-4-carbonyl]-amino}-methyl)-phenyl]-essigsäureethylester

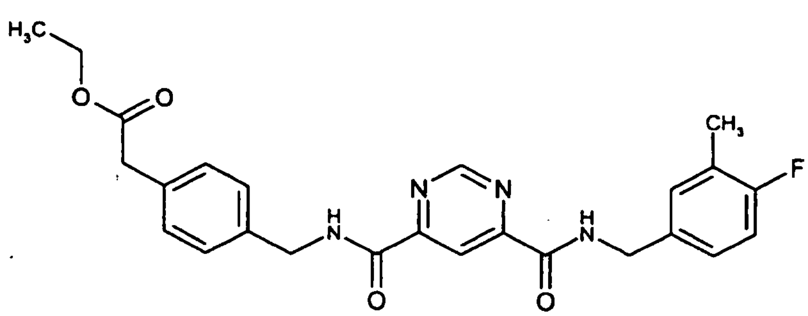

**a)** 6-(4-Fluoro-3-methyl-benzylcarbamoyl)-pyrimidin-4-carbonsäuremethylester

8,81 g (0,045 mol) Pyrimidin-4,6-dicarbonsäuredimethylester wurden in 200 ml DMF gelöst und mit 6,25 g (0,045 mol) 4-Fluoro-3-methyl-benzylamin versetzt und 48 Stunden (h) bei 60°C gerührt. Das Lösungsmittel wurde im Vakuum entfernt und der Rückstand in Essigsäureethylester aufgenommen. Die organische Phase wurde mit gesättigter Natriumhydrogencarbonatlösung und 0,5 N HCl gewaschen und getrocknet (MgS04). Nach Abfiltrieren und Einengen des Lösungsmittels im Vakuum wurde der Rückstand in Isopropanol verrührt. Man erhielt 8,75 g Produkt, das ohne weitere Reinigung weiter umgesetzt wurde.

**b)** 6-(4-Fluoro-3-methyl-benzylcarbamoyl)-pyrimidin-4-carbonsäure

8,75 g (0,02 mol) 6-(4-Fluoro-3-methyl-benzylcarbamoyl)-pyrimidin-4-carbonsäuremethylester (70%) wurden in 150 ml Ethanol aufgenommen und mit 1,89 g (0,022 mol) NaOH in 6 ml Wasser versetzt. Nach 3 Stunden (h) bei Raumtemperatur wurde das Lösungsmittel unter vermindertem Druck entfernt, der Rückstand mit Wasser versetzt und mit konz. HCl auf pH < 2 gestellt. Der Niederschlag wurde abgesaugt und getrocknet. Man erhielt 5,5 g (94%) 6-(4-Fluoro-3-methyl-benzylcarbamoyl)-pyrimidin-4-carbonsäure. MS ($ES^+$): m/e= 289.09

**c)** (4-Aminomethyl-phenyl)-essigsäureethylester

0,5 g (2,6 mmol) (4-Cyano-phenyl)-essigsäureethylester wurden in 70 ml ethanolischer Ammoniaklösung gelöst und über Raney-Nickel bei Raumtemperatur und Normaldruck hydriert. Nach 45 Minuten wurde abfiltriert und eingeengt. Man erhielt 0,42 g (82%) (4-Aminomethyl-phenyl)-essigsäureethylester. MS ($ES^+$): m/e= 194.11

**d)** [4-({[6-(4-Fluoro-3-methyl-benzylcarbamoyl)-pyrimidin-4-carbonyl]-amino}-methyl)-phenyl]- essigsäureethylester

1,3 g (4,5 mmol) 6-(4-Fluoro-3-methyl-benzylcarbamoyl)-pyrimidin-4-carbonsäure und 1,042 g ((5,4 mmol) (4-Aminomethyl-phenyl)-essigsäureethylester wurden in 30 ml DMF gelöst und mit 1,02 g (4,9 mmol) Dicyclohexylcarbodiimid und 0,607 g (4,5 mmol) Hydroxybenzotriazol bei 5°C versetzt. Es wurde 5 (h) Stunden gerührt und abgesaugt. Das Lösungsmittel wurde im Vakuum entfernt, der Rückstand in Essigsäureethylester aufgenommen und mit gesättigter wässriger NaHC03 Lösung gewaschen. Die organische Phase wurde getrocknet (MgSO4), abfiltriert und unter vermindertem Druck eingeengt. Man erhielt 2,66 g Produkt, das über präparative HPLC weiter aufgereinigt wurde. MS ($ES^+$): m/e= 464.19

**Referenz Beispiel 2:**

[4-({[6-(4-Fluoro-3-methyl-benzylcarbamoyl)-pyrimidin-4-carbonyl]-amino}-methyl)-phenyl]-essigsäure

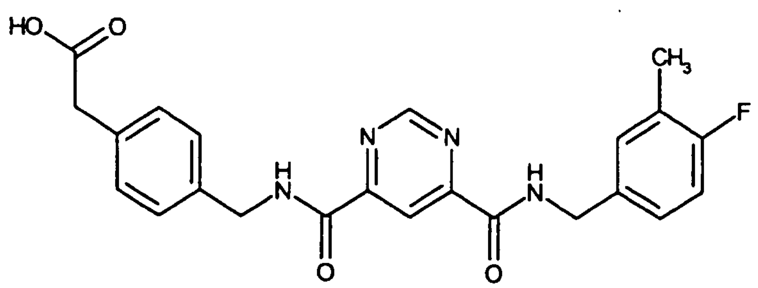

2,4 g (5,2 mmol) [4-({[6-(4-Fluoro-3-methyl-benzylcarbamoyl)-Pyrimidin-4-carbonyl]-amino}-methyl)-phenyl]-essigsäureethylester wurden in 150 ml Wasser aufgenommen und mit 10 ml Wasser und 0,227 g (5,7 mmol) NaOH versetzt. Nach 5 Tagen Rühren bei Raumtemperatur wurde das Lösungsmittel unter reduziertem Druck entfernt und der Rückstand mit Ethanol verrührt und abfiltriert. Man erhielt 1,51 g (67%) [4-({[6-(4-Fluoro-3-methyl-benzylcarbamoyl)-pyrimidin-4-carbonyl]-amino}-methyl)-phenyl]- essigsäure. MS ($ES^+$): m/e= 436.15

**Beispiel 3**

Pyrimidin-4,6-dicarbonsäure 4-(4-diethylcarbamoyl-benrylamid) 6-(3-methoxy-benzylamid)

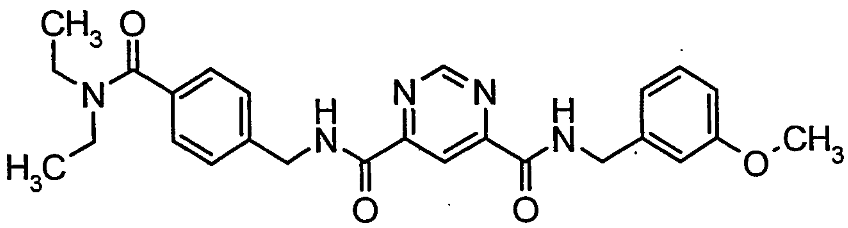

**a)** Synthese von 6-(3-Methoxy-benzylcarbamoyl)-pyrimidin-4-carbonsäure

26 g (88 mmol) 6-(3-Methoxy-benzylcarbamoyl)-pyrimidin-4-carbonsäuremethylester (hergestellt durch Umsetzung von Pyrimidin-4,6-dicarbonsäuredimethylester mit 3-Methoxybenzylamin) wurden in 100 ml Tetrahydrofuran gelöst und mit 104 ml (1,2 Äquivalente) einer 1 molaren wässrigen Lithiumhydroxid-Lösung versetzt und die. Reaktionsmischung wurde anschließend 18 Stunden bei Zimmertemperatur gerührt.

Anschließend wurde der Großteil des verwendeten Lösungsmittels unter reduziertem Druck abdestilliert, der Rückstand von unlöslichen Nebenprodukten abfiltriert und das Filtrat mit 20%iger wässriger Zitronensäurelösung angesäuert. Dabei kristallisierte 6-(3-Methoxy-benzyl-carbamoyl)-pyrimidin-4-carbonsäure in Form hellgelber Kristalle aus, welche abfiltriert wurden.

Man erhielt 19 g (66,2 mmol) 6-(3-Methoxy-benzylcarbamoyl)-pyrimidin-4-carbonsäure (Ausbeute 75 % d. Theorie; MS ($ES^+$): m/e = 287,8)

**b)** 4-({[6-(3-Methoxy-benzylcarbamoyl)-pyrimidin-4-carbonym]-amino}-methyl)-benzoesäuremethyl ester

4,3 g 6-(3-Methoxy-benrylcarbamoyl)-pyrimidin-4-carbonsäure (15 mmol) aus a) wurde in 50 ml absolutem N, N-Dimethylformamid gelöst und unter Rühren bei 0 ˚C nacheinander mit 3,3 g (16,5 mmol) Methyl-4-(aminomethyl)-benzoat hydrochlorid, 5,4 g (16,5 mmol) 0-[(Cyan-ethoxycarbonylmethylen)-amino]-N,N,N',N'-tetramethyluronium-tetrafluoroborat (TOTU) und 4,6 ml Triethylamin (33 mmol) versetzt. Das Reaktionsgemisch wurde 1 Stunde bei 0 ˚C und anschließend 12 Stunden bei Zimmertemperatur gerührt.

Zur Aufarbeitung wurde das Lösungsmittel unter reduziertem Druck abdestilliert und der Rückstand mit 100 ml Dichlormethan aufgenommen. Die organische Phase wurde mit 100 ml gesättigter wässriger Natriumhydrogencarbonat-Lösung und dann dreimal mit jeweils 100 ml Wasser gewaschen. Nach Trocknung der organischen Phase mittels $Na_2SO_4$ wurde das Solvens unter reduziertem Druck abdestilliert. Der ölige Rückstand wurde mit wenig Diethylether verrieben, wobei farblose Kristalle auskristallisierten. Nach Abfiltration des Reaktionsproduktes und Waschen mittels n-Pentan erhielt man 6,6 g 4-({[6-(3-Methoxy-benzylcarbamoyl)-pyrimidin-4-carbonyl]-amino}-methyl)-benzoesäuremethylester (hellgelbe Kristalle). Das Reaktionsprodukt besitzt laut LC-MS-Analyse eine Reinheit von 88% (MS ($ES^+$): m/e = 435,2).

**c)** 4-({[6-(3-Methoxy-benzylcarbamoyl)-pyrimidin-4-carbonyl]-amino}-methyl)-benzesäure

6,6 g des in b) hergestellten Methylesters wurden in 100 ml Tetrahydrofuran gelöst und mit 36 ml (2,4 Äquivalente) einer 1 molaren Lithiumhydroxid-Lösung versetzt und die Reaktionsmischung wurde anschließend 4 Stunden unter Rückfluss des Lösungsmittels gerührt.

Anschließend wurde das Lösungsmittel unter reduziertem Druck abdestilliert. Nachdem mit 50 ml Wasser versetzt wurde, wurde über Celite® Filterhilfe abfiltriert und das Filtrat wurde mit 2 n wässriger Salzsäure angesäuert. Das Reaktionsprodukt fiel beim Ansäuern aus und wurde abfiltriert.

Man erhielt 3,05 g 4-({[6-(3-Methoxy-benzylcarbamoyl)-pyrimidin-4-carbonyl]-amino}-methyl)-benzoesäure, hellgelbe Kristalle [Ausbeute 48 % d. Th.; MS ($ES^+$): m/e = 421,31]

**d)** Pyrimidin-4,6-dicarbonsäure 4-(4-diethylcarbamoyl-benzylamid) 6-(3-methoxybenzylamid)

420 mg 4-({[6-(3-Methoxy-benzylcarbamoyl)-pyrimidin-4-carbonyl]-amino}-methyl)-benzoesäure aus c) wurden in 5 ml absolutem N,N-Dimethylformamid gelöst und unter Rühren bei 0 ˚C nacheinander mit 115 μl Diethylamin, 361 mg O-[(Cyan-ethoxycarbonylmethylen)-amino]-N,N,N',N'-tetramethyluroniumtetrafluoroborat (TOTU) und 153 μl Triethylamin versetzt und das Reaktionsgemisch wurde 1 Stunde bei 0 ˚C und anschließend 12 Stunden bei Zimmertemperatur gerührt.

Zur Aufarbeitung wurde das Lösungsmittel unter reduziertem Druck abdestilliert und der Rückstand mit 100 ml Dichlormethan aufgenommen. Die organische Phase wurde mit 30 ml gesättigter wässriger Natriumhydrogencarbonat-Lösung und dann dreimal mit jeweils 30 ml Wasser gewaschen. Nach Trocknung der organischen Phase mittels $Na_2SO_4$ wurd das Solvens unter reduziertem Druck abdestilliert. Der ölige Rückstand wurde mittels Säulenchromatographie an Kieselgel (40 - 63 μ) mit Ethylacetat/n-Heptan, Mischungsverhältnis 2:1, als mobiler Phase gereinigt. Nach Abdestillieren des Lösungsmittels erhielt man einen öligen Rückstand, welcher nach Zugabe von wenig Diethylether langsam kristallisierte.

Man erhielt 270 mg Pyrimidin-4,6-dicarbonsäure 4-(4-diethylcarbamoyl-benzylamid) 6-(3-methoxy-benzylamid, farblose Kristalle (Ausbeute 57 % d. Th. [MS ($ES^+$): m/e = 476,40]).

**Referenz Beispiel 62**

Pyrimidin-4,6-dicarbonsäure 4-[(2,3-dihydro-benzofuran-5-ylmethyl)-amid] 6-[(pyridin-4-ylmethyl)-amid]

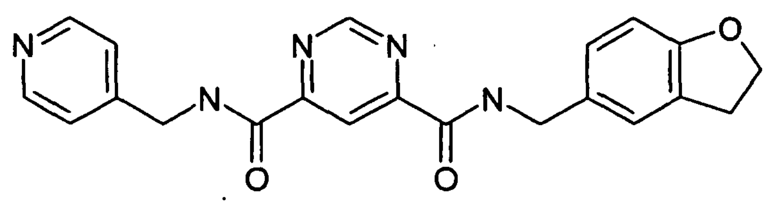

**a)** Synthese von 6-[(Pyridin-4-ylmethyl)-carbamoyl]-pyrimidin-4-carbonsäure

9,7 g (35,7 mmol) 6-[(Pyridin-4-ylmethyl)-carbamoyl]-pyrimidin-4-carbonsäure-methylester (hergestellt durch Umsetzung von Pyrimidin-4,6-dicarbonsäuredimethylester mit Pyridin-4-yl-methylamin wurden in 80 ml Tetrahydrofuran und 40 ml Wasser gelöst, mit 40 ml einer 1 molaren wässrigen NaOH-Lösung versetzt und die Reaktionmischung anschließend 2 Stunden bei Zimmertemperatur gerührt.

Zur Aufarbeitung wurde das Reaktionsgemisch am Rotationsverdampfer unter reduziertem Druck auf die Hälfte des ursprünglichen Volumens eingeengt. Anschließend wurde mit 22 ml wässriger 2 n Salzsäure-Lösung angesäuert und das Reaktionsgemisch bis zur zur Trockne am Rotationsverdampfer weiter eingeengt.

Man erhielt 12,2 g farbloses Festprodukt, welches direkt weiter entsprechend **62 b)** umgesetzt wurde.

**b)** Pyrimidin-4,6-dicarbonsäure 4-[(2,3-dihydro-benzofuran-5-ylmethyl)-amid] 6-[(pyridin-4-ylmethyl)-amid]

12, 2 g der unter **62a)** hergestellten Verbindung wurden in 150 ml absolutem DMF gelöst und unter Rühren bei 0˚C (Eiskühlung) nacheinander mit 6,63 g (35,7 mmol) 5-Aminomethyl-2,3-dihydrobenzofuranhydrochlorid, 11,7 g (35,7 mmol) 0-[(Cyan-ethoxycarbonylmethylen)-amino]-N,N,N',N'-tetramethyluronium-tetrafluoroborat (TOTU) und 20 ml Triethylamin versetzt. Das Reaktionsgemisch wurde nach beendeter Zugabe 1 Stunde bei 0˚C und 4 Stunden bei Zimmertemperatur gerührt.

Zur Aufarbeitung wurde das Solvens unter reduziertem Druck abdestilliert und der Rückstand mit 200 ml Dichlormethan aufgenommen. Die organische Phase wurde anschließend zweimal mit gesättigter wässriger Natriumhydrogencarbonat-Lösung und einmal mit Wasser gewaschen, mit Natriumsulfat getrocknet und das Solvens unter reduziertem Druck am Rotationsverdampfer entfernt. Das als öliger Rückstand anfallende Reaktionsprodukt kristallisierte nach Zugabe von wenig Diethylether in Form rosafarbener Kristalle aus. Zur weiteren Reinigung wurde zweimal aus jeweils 200 ml Isopropanol umkristallisiert.

Man erhielt 10g (25,6 mmol)Pyrimidin-4,6-dicarbonsäure 4-[(2,3-dihydro-benzofuran-5-ylmethyl)-amid] 6-[(pyridin-4-ylmethyl)-amid] (Ausbeute 72 % d. Theorie, bezogen auf beide Reaktionsstufen; MS ($ES^+$): 390,08)

1H-NMR (400 MHz, $d_6$-DMSO): $\delta$ = 3.13 (t, J = 8.6 Hz,2H), 4.42 (d, J = 6.4 Hz, 2H), 4.48 (t, J = 8.6 Hz, 2 H), 4.54 (d, J = 6.4 Hz, 2 H), 6.69 (d, J = 8 Hz, 1 H), 7.07 (m, 1 H), 7.22 (m, 1 H), 7.31 (m, 2 H), 8.46 (m, 1 H), 8.50 (m 2 H), 9.47 (m, 1 H), 9.58 (t, J = 6.4 Hz, 1 H), 9.80 (t, J = 6.4 Hz, 1 H)

**Beispiel 117**

Pyrimidin-4,6-dicarbonsäure 4-[(2,3-dihydro-benzofuran-5-ylmethyl)-amid] 6-[4-(morpholin-4-carbonyl)-benzylamid]

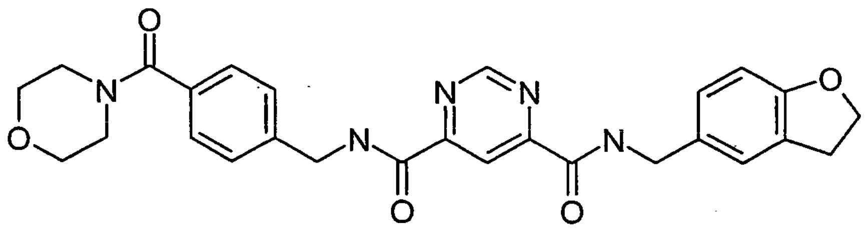

**a)** Synthese von 6-[(2,3-Dihydro-benzofuran-5-ylmethyl)-carbamoyl]-pyrimidin-4-carbonsäure

16,1 g (51 mmol) 6-[(2,3-Dihydro-benzofuran-5-ylmethyl)-carbamoyl]-pyrimidin-4-carbonsäuremethylester (hergestellt durch Umsetzung von Pyrimidin-4,6-dicarbonsäuredimethylester mit 5-Aminomethyl-2,3-dihydrobenzofuran) wurden in 150 ml Tetrahydrofuran gelöst, mit 62 ml einer 1 molaren wässrigen LiOH-Lösung versetzt und die Reaktionmischung anschließend 2 Stunden bei Zimmertemperatur gerührt.
Zur Aufarbeitung wurde das Reaktionsgemisch am Rotationsverdampfer unter reduziertem Druck eingeengt. Anschließend wurde das Rohprodukt mit 100 ml Wasser aufgenommen und nach Zusatz von Aktivkohle über Celite© Klärschicht filtriert. Die erhaltene Mutterlauge wurde anschließend mittels Zusatz von 2n wässriger HCl-Lösung angesäuert, wobei das Reaktionsprodukt langsam in Form farbloser Kristalle ausfällt.
Nach Abfiltration und Trocknung des Reaktionsproduktes erhielt man 8.3 g (27 mmol) farbloses Festprodukt, welches direkt weiter zu **117b** umgesetzt wurde; Ausbeute 53 % d. Theorie.
MS ($ES^+$): 300,1

**b)** Synthese von 4-[({6-[(2,3-Dihydro-benzofuran-5-ylmethyl)-carbamoyl]-pyrimidin-4-carbonyl}-amino)-methyl]-benzoesäuremethyl ester

4,7 g (15,7 mmol) der unter a) hergestelltenVerbindung wurden in 30 ml absolutem DMF gelöst und unter Rühren bei 0˚C nacheinander mit 3,5 g (17,3 mmol) 4-Amino-methylbenzoesäure-methylester, 5,7 g (17,3 mmol) O-[(Cyan-ethoxy-carbonyl-methylen)-amino]-N,N,N',N'-tetramethyluronium-tetrafluoroborat (TOTU) und 4,8 ml Triethylamin versetzt. Das Reaktionsgemisch wurde nach beendeter Zugabe 1 Stunde bei 0˚C und 8 Stunden bei Zimmertemperatur gerührt.
Zur Aufarbeitung wurde das Solvens unter reduziertem Druck abdestilliert und der Rückstand mit 100 ml Dichlormethan aufgenommen. Die organische Phase wurde anschließend zweimal mit gesättigter wässriger Natriumhydrogencarbonat-Lösung und einmal mit Wasser gewaschen, mit Natriumsulfat getrocknet und das Solvens unter reduziertem Druck am Rotationsverdampfer entfernt. Das als öliger Rückstand anfallende Reaktionsprodukt kristallisierte nach Zugabe von wenig Diethylether in Form hellgelber Kristalle aus.
Man erhielt 6,8 (15,2 mmol) 4-[({6-[(2,3-Dihydro-benzofuran-5-ylmethyl)-carbamoyl]-pyrimidin-4-carbonyl}-amino)-methyl]-benzoesäuremethyl ester, Ausbeute 97 % d. Theorie.
MS ($ES^+$): 447,1

**c)** 4-[({6-[(2,3-Dihydro-benzofuran-5-ylmethyl)-carbamoyl]-pyrimidin-4-carbonyl}-amino)-methyl]-benzoesäure

6,28 g (14 mmol) 4-[({6-[(2,3-Dihydro-benzofuran-5-ylmethyl)-carbamoyl]-pyrimidin-4-carbonyl}-amino)-methyl]-benzoesäuremethylester (siehe **117b**) wurden in 150 ml Tetrahydrofuran und 70 ml Wasser suspendiert und mit 16,9 ml einer 1 n wässrigen NaOH-Lösung versetzt und die Reaktionmischung anschließend 24 Stunden bei Zimmertemperatur gerührt.
Zur Aufarbeitung wurde das Reaktionsgemisch am Rotationsverdampfer unter reduziertem Druck auf ein Volumen von ca. 50 ml eingeengt und mit 100 ml Eiswasser versetzt.
Anschließend wurde mittels 2 n wässriger HCl-Lösung angesäuert, wobei das Reaktionsprodukt in Form hellgelber Kristalle ausfällt.

Nach Abfiltration, Waschen mit wenig Wasser und Trocknung des Reaktionsproduktes erhielt man 5.4 g (12.5 mmol) farbloses Festprodukt, welches direkt weiter zu 117d umgesetzt wurde; Ausbeute 89 % d. Theorie. MS ($ES^+$): 433,2
1 H-NMR (400 MHz, $d_6$-DMSO): δ = 3.13 (t, J = 8.7 Hz, 2 H), 4.43 (d, J = 6.1 Hz, 2 H), 4.48 (t, J = 8.7 Hz, 2 H), 4.59 (d, J = 6.3 Hz, 2 H), 6,69 (d, J = 8.1 Hz, 1 H), 7.07 (m, 1 H), 7.22 (m, 1H), 7.44 (m, 2 H), 7.88 (m, 1 H), 7.90 (m, 1 H), 8.47 (d, J = 1.5 Hz, 1 H), 9.46 (d, J = 1.3 Hz, 1 H), 9.56 (t, J = 6.3 Hz, 1 H), 9.76 (t, J = 6.3 Hz, 1 H), 12.90 (br s, 1H)

**d)** Pyrimidin-4,6-dicarbonsäure 4-[(2,3-dihydro-benzofuran-5-ylmethyl)-amid] 6-[4-(morpholin-4-carbonyl)-benzylamid]

432mg (1 mmol) der unter c) hergestelltenVerbindung wurden in 5 ml absolutem DMF gelöst und unter Rühren bei 0˚C nacheinander mit 96 μl (1,1 mmol) Morpholin, 361 mg (1,1 mmol). O-[(cyan-ethoxy-carbonyl-methylen)-amino]-N,N,N',N'-tetramethyluronium-tetrafluoroborat (TOTU) und 155 μl Triethylamin versetzt. Das Reaktionsgemisch wurde nach beendeter Zugabe 1 Stunde bei 0˚C und 8 Stunden bei Zimmertemperatur gerührt.

Zur Aufarbeitung wurde das Solvens unter reduziertem Druck abdestilliert und der Rückstand mit 30 ml Dichlormethan aufgenommen. Die organische Phase wurde anschließend zweimal mit gesättigter wässriger Natriumhydrogencarbonat-Lösung und einmal mit Wasser gewaschen, mit Natriumsulfat getrocknet und das Solvens unter reduziertem Druck am Rotationsverdampfer entfernt. Das als öliger Rückstand anfallende Rohprodukt wurde mittels Chromatographie an Kieselgel (40 - 63 μ; mobile Phase: Ethylacetat/Methanol = 20/1) gereinigt. Nach Entfernen der mobilen Phase mittels Destillation unter reduziertem Druck erhielt man ein öliges Reaktionsprodukt, welches nach Versetzen mit Diethylether in Form farbloser Kristalle auskristallisierte.

Man erhielt 340 mg (068 mmol) farblose Kristalle, Ausbeute 68 % d. Theorie.
MS ($ES^+$): 502,27
1 H-NMR (500 MHz, $d_6$-DMSO): δ = 2.60 (m, 2 H), 3,4-3.6 (br m, 8 H), 4.42 (d, J = 6.5 Hz, 2 H), 4.48 (t, J = 8.6 Hz, 2 H), 4.56 (d, J = 6.5 Hz, 2 H), 6.68 (d, J = 8.3 Hz, 1H), 7.07 (d, J = 6.5 Hz, 1 H), 7.22 (s, 1 H), 7.88 (m, 4 H), 8.46 (m, 1 H), 9.46 (m, 1 H), 9.57 (t, J = 6.5 Hz, 1 H), 9.75 (t, J = 6.5 Hz, 1 H)

Die nachfolgenden Verbindungen wurden in vergleichbarer Weise hergestellt.

Tabelle 1:

| Beispiel | Struktur | MS (ESI+) |
|---|---|---|
| 4 | 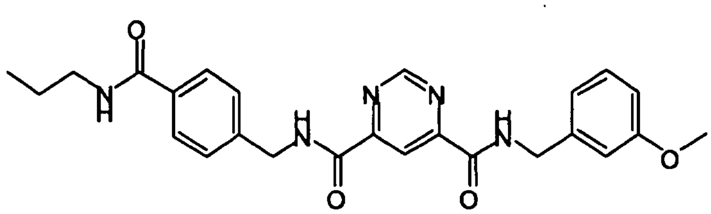 | 462,36 |
| 5 | 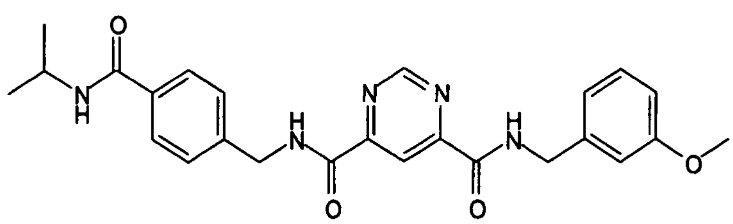 | 462,37 |

(fortgesetzt)

| Beispiel | Struktur | MS (ESI+) |
|---|---|---|
| 9 | 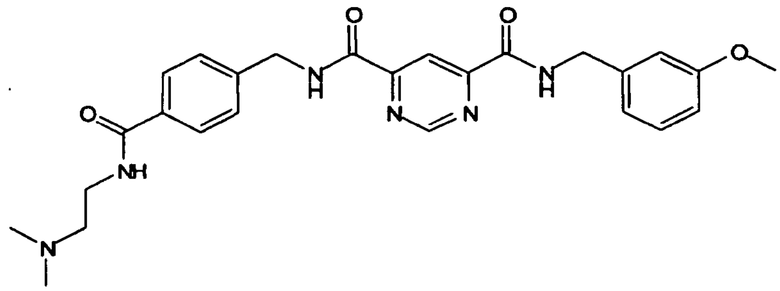 | 491,38 |
| 10 | 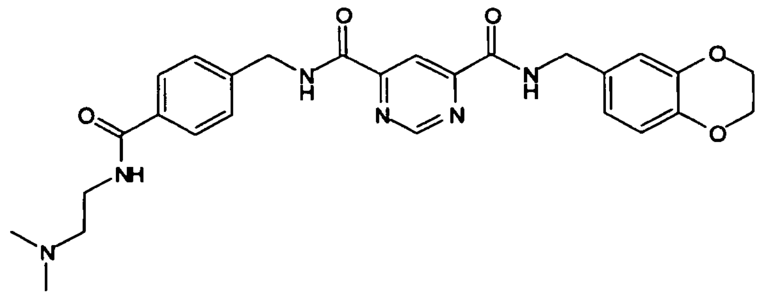 | 519,36 |
| 11 | 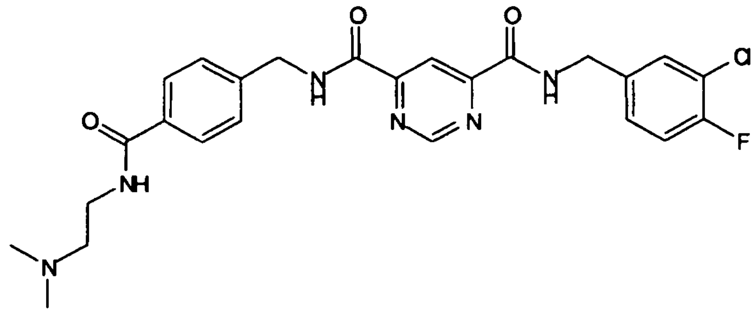 | 513,31 |
| 19 | 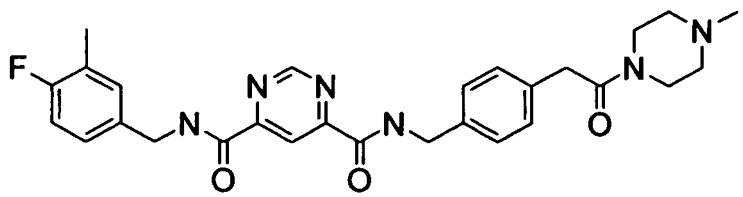 | 519,48 |
| 22 | 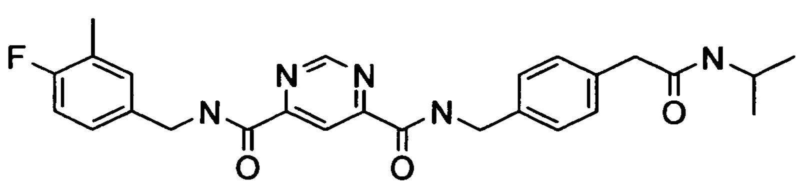 | 478,44 |

(fortgesetzt)

| Beispiel | Struktur | MS (ESI+) |
|---|---|---|
| 23 | 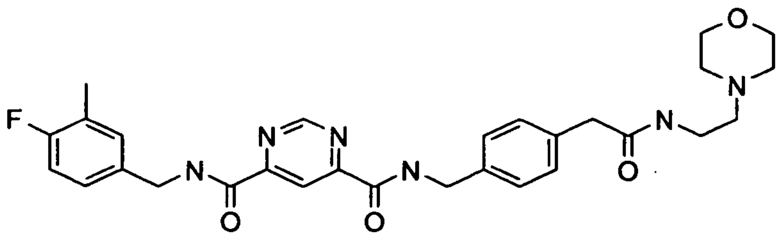 | 549,50 |
| 24 | 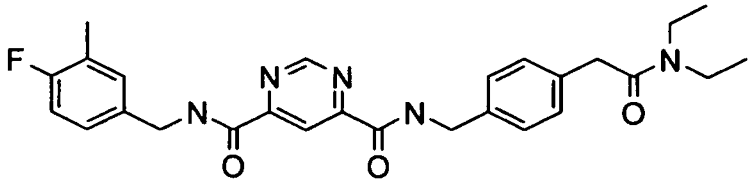 | 492,45 |
| 25 | 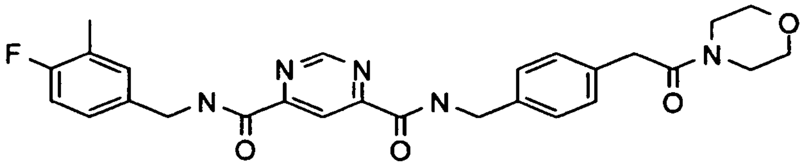 | 506,45 |
| 36 | 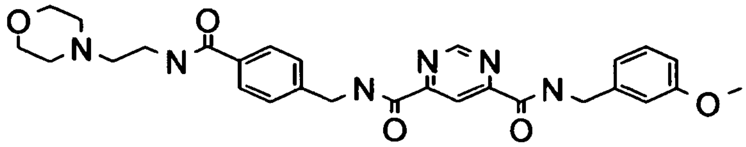 | 533,30 |
| 37 | 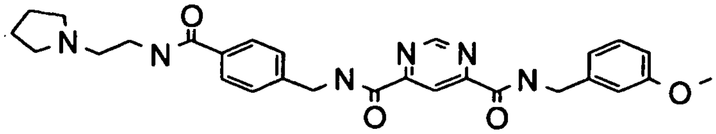 | 517,27 |
| 70 | 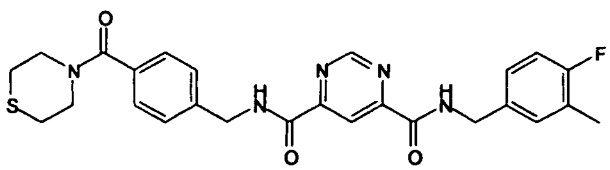 | 508,16 |
| 71 | 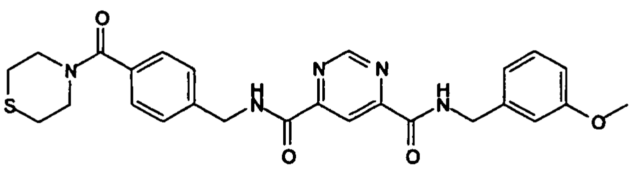 | 506,09 |
| 72 | 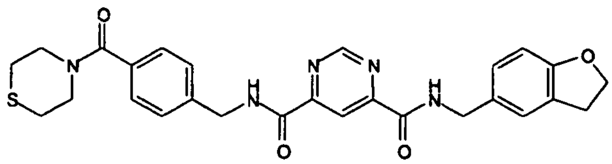 | 518,1 |

(fortgesetzt)

| Beispiel | Struktur | MS (ESI+) |
|---|---|---|
| 78 | 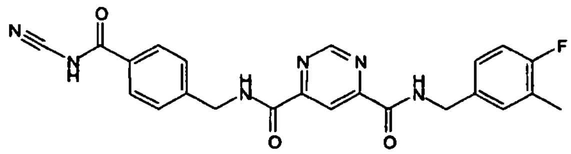 | 464,12 |
| 79 | 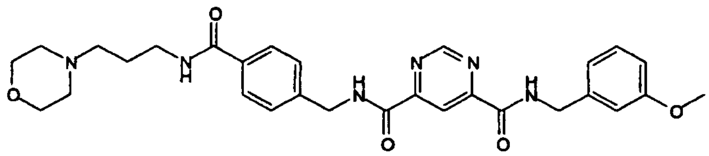 | 547,16 |
| 80 | 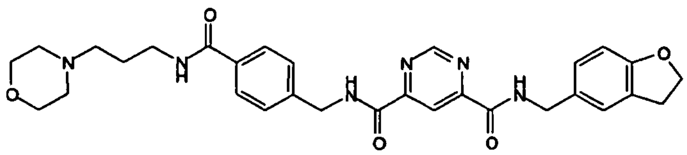 | 559,18 |
| 81 | 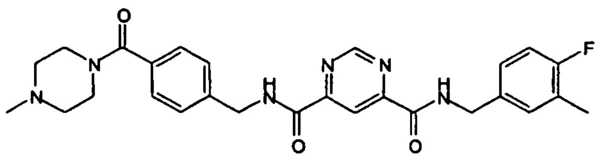 | 505,13 |
| 82 | 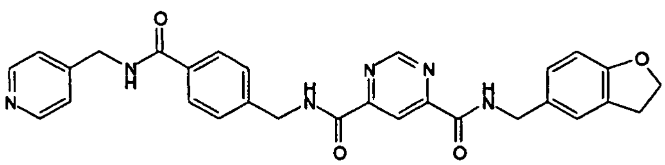 | 523,15 |
| 85 | 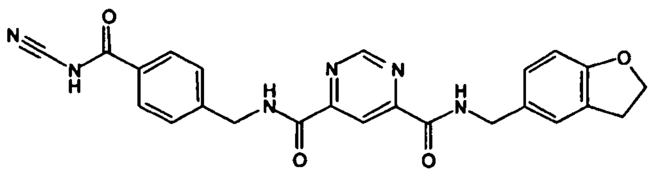 | 457,21 |
| 86 | 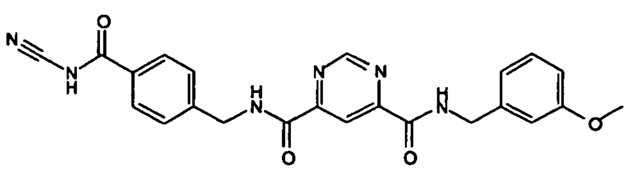 | 445,13 |
| 87 | 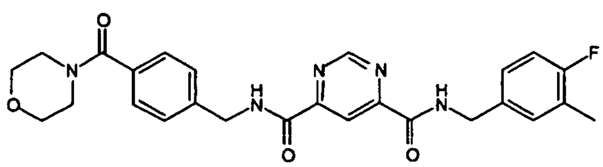 | 492, 39 |

(fortgesetzt)

| Beispiel | Struktur | MS (ESI+) |
|---|---|---|
| 92 | 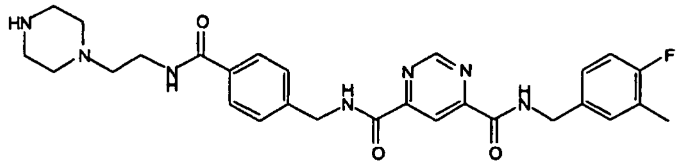 | 534,21 |
| 96 | 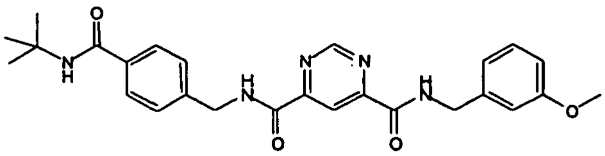 | 476,27 |
| 97 | 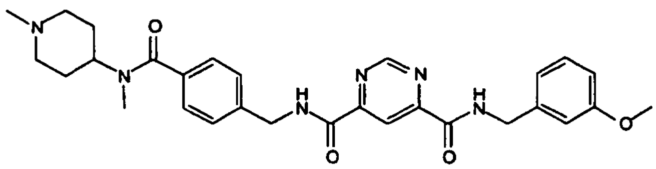 | 531,31 |
| 99 | 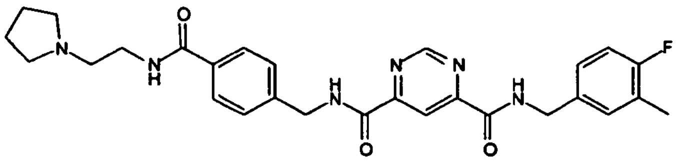 | 519,28 |
| 102 | 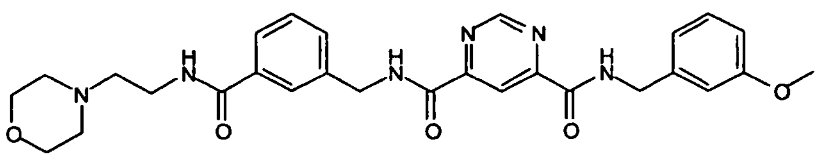 | 533,23 |
| 105 | 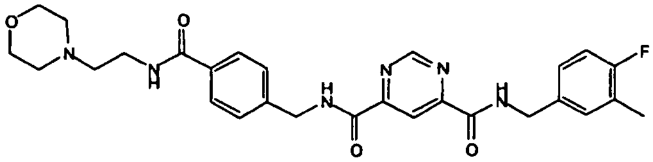 | 535,25 |
| 107 | 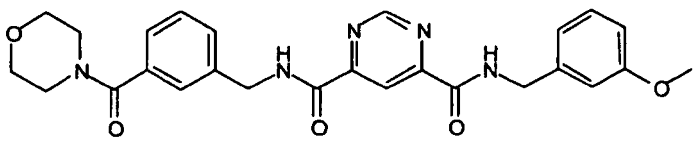 | 490,25 |
| 108 | 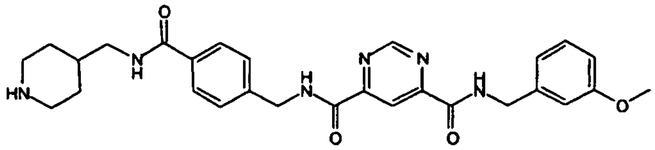 | 517,28 |

(fortgesetzt)

| Beispiel | Struktur | MS (ESI+) |
|---|---|---|
| 109 | 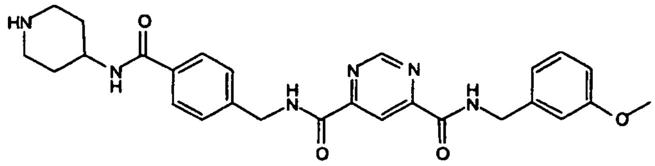 | 503,28 |
| 110 | 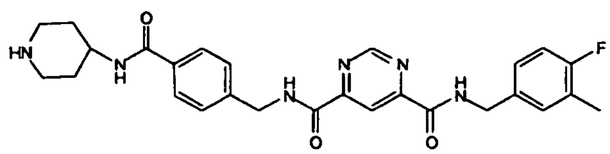 | 505,23 |
| 111 | 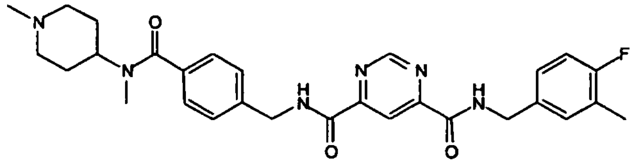 | 533,26 |
| 113 | 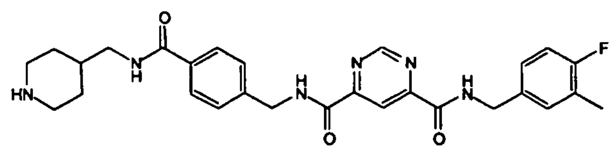 | 519,27 |
| 114 | 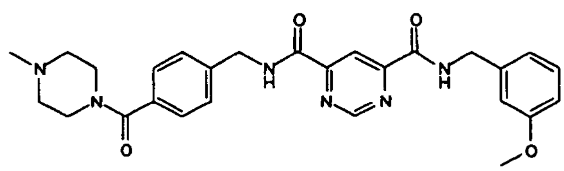 | 503,24 |
| 117 | 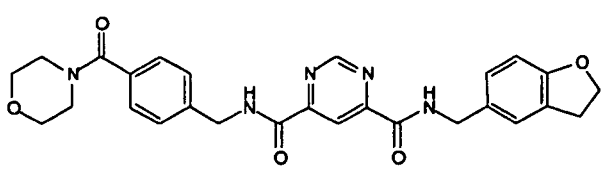 | 502,27 |
| 119 | 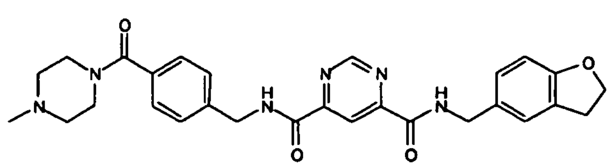 | 515,36 |
| 120 | 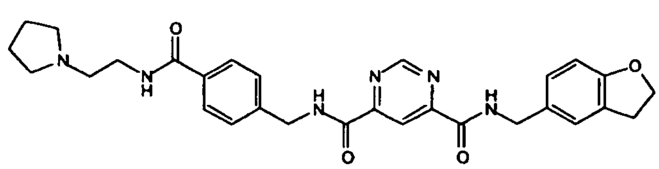 | 529,29 |

(fortgesetzt)

| Beispiel | Struktur | MS (ESI+) |
|---|---|---|
| 122 | 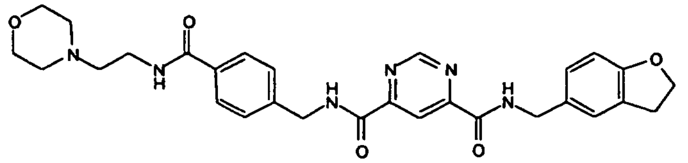 | 545,24 |
| 150a | 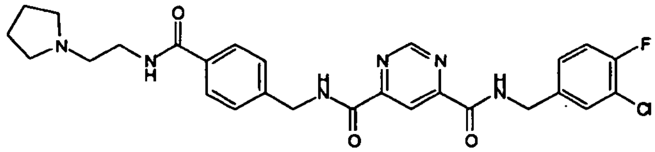 | 539,4 |
| 151 | 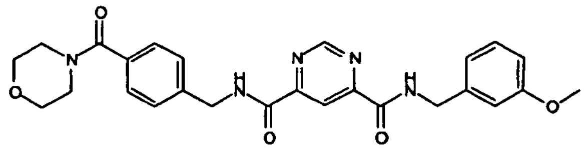 | 490,43 |
| 152 | 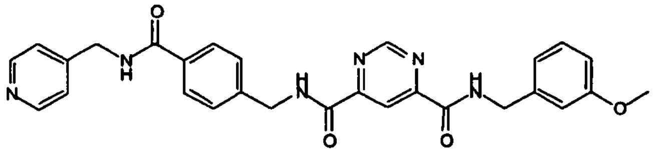 | 511,41 |
| 153 | 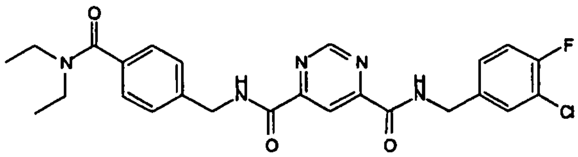 | 498,36 |
| 154 | 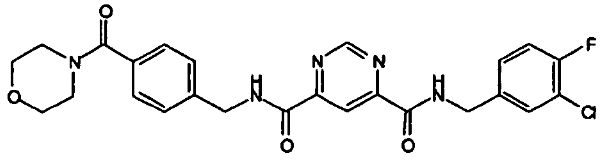 | 512, 37 |
| 155 | 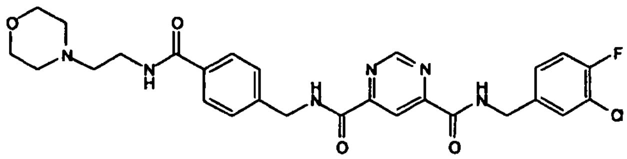 | 555,4 |
| 156 | 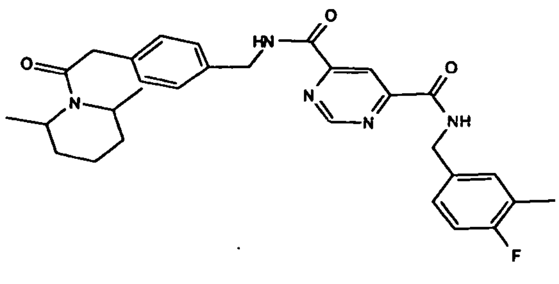 | 532,46 |

(fortgesetzt)

| Beispiel | Struktur | MS (ESI+) |
|---|---|---|
| 158 | 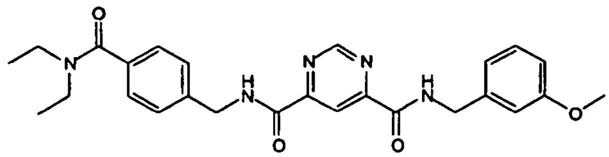 | 476,4 |

Pharmkologische Beispiele

Bestimmung der enzymatischen Aktivität der katalytischen Domäne der humanen Kollagenase -3 (MMP-13).

Dieses Protein wird als inaktives Pro-Enzym von der Fa. INVITEK, Berlin, erhalten (Katalog Nr. 30 100 803). Aktivierung des Proenzyms:

2 Volumenanteile Proenzym werden mit 1 Volumenanteil APMA-Lösung bei 37 ˚C für 1,5 Stunden inkubiert. Die APMA-Lösung wird aus einer 10 mmol/L p-Aminophenyl-Mercuric Acetate Lösung in 0,1 mmol/L NaOH durch Verdünnen mit 3 Volumenteile Tris/HCl Puffer pH7,5 (siehe unten) hergestellt. Der pH-Wert wird durch Zugabe von 1mmol/L HCl zwischen 7,0 und 7,5 eingestellt. Nach der Aktivierung des Enzyms wird dieses mit dem Tris/HCl Puffer auf eine Konzentration von 1,67 µg/mL verdünnt.

Zur Messung der Enzymaktivität werden 10 µL Enzymlösung mit 10 µL einer 3%igen (v/v) gepufferten Dimethylsulfoxid-Lösung (Reaktion 1) für 15 Minuten inkubiert. Zur Messung der Enzyminhibitoraktivität werden 10 µL Enzymlösung mit 10 µL einer 3%igen (v/v) gepufferten Dimethylsulfoxid-Lösung, die den Enzyminhibitor enthält, inkubiert (Reaktion 2).

Sowohl bei Reaktion 1 als auch bei Reaktion 2 wird nach Zugabe von 10 µL einer 3%igen (v/v) wässrigen Dimethylsulfoxid-Lösung, die 0,75 mmol/L des Substrates enthält, die Enzymreaktion fluoreszenzspektroskopisch verfolgt (328 nm (Extinktion)/ 393 nm(Emission)).

Die Enzymaktivität wird dargestellt als Extinktionszunahme/Minute.
Die Inhibitorwirkung wird als prozentuale Hemmung nach folgender Formel berechnet:

$$\% \text{ Hemmung} = 100 - [(\text{Extinktionszunahme/Minute in Reaktion 2}) / (\text{Extinktionszunahme/Minute in Reaktion 1}) \times 100].$$

Der $IC_{50}$, d.h. die für eine 50%ige Hemmung der Enzymaktivität erforderliche Inhibitorkonzentration wird grafisch durch Auftragen der prozentualen Hemmungen bei verschiedenen Inhibitorkonzentrationen ermittelt.

Die Pufferlösung enthält 0,05% Brij (Sigma, Deisenhofen, Deutschland) sowie 0,1 mol/L Tris/HCl, 0,1 mol/L NaCl, 0,01 mol/L $CaCl_2$ (pH=7,5).
Die Enzymlösung enthält 1,67 µg/mL der Enzymdomäne.
Die Substratlösung enthält 0,75 mmol/L des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-$NH_2$ (Bachem, Heidelberg, Deutschland).

Die nachfolgende Tabelle 2 zeigen die Ergebnisse.

Tabelle 2:

| Beispiel | $IC_{50}$ MMP13 (nM) | Beispiel | $IC_{50}$ MMP13 (nM) | Beispiel | $IC_{50}$ MMP13 (nM) | Beispiel | $IC_{50}$ MMP13 (nM) |
|---|---|---|---|---|---|---|---|
| 3 | 4 | 78 | 3 | 105 | 23 | 151 | 9 |
| 4 | 13 | 82 | 30 | 109 | 50 | 153 | 15 |
| 36 | 10 | 85 | 3,5 | 114 | 50 | 154 | 22 |
| 70 | 24 | 86 | 2,5 | 117 | 8 | 158 | 4 |

(fortgesetzt)

| Beispiel | $IC_{50}$ MMP13 (nM) | Beispiel | $IC_{50}$ MMP13 (nM) | Beispiel | $IC_{50}$ MMP13 (nM) | Beispiel | $IC_{50}$ MMP13 (nM) |
|---|---|---|---|---|---|---|---|
| 71 | 9 | 87 | 24 | 119 | 35 | | |
| 72 | 10 | 96 | 50 | 122 | 43 | | |

Bestimmung der enzymatischen Aktivität der katalytischen Domäne der humanen Neutrophilen-Kollagenase (MMP-8) und des humanen Stromelysins (MMP-3).

Die Enzyme humane Neutrophilen-Kollagenase und humanes Stromelysin wurden wie in Weithmann et al Inflamm Res, 46 (1997), Seiten 246-252, beschrieben, als aktive katalytische Domänen hergestellt, durchgeführt. Die Messung der Enzymaktivität sowie die Bestimmung der inhibitorischen Wirkung von Hemmstoffen auf die Enzymaktivität erfolgte ebenfalls wie dort beschrieben.

Die Verbindungen gemäß der obengenannten Beispiele zeigten bei der Bestimmung der humanen Neutrophilen-Kollagenase und des humanen Stromelysin jeweils IC50-Werte von mehr als 100000 nM. Damit sind diese Verbindungen praktisch unwirksam bei der Inhibition von MMP 3 und 8.

**Patentansprüche**

**1.** Verbindung der Formel I

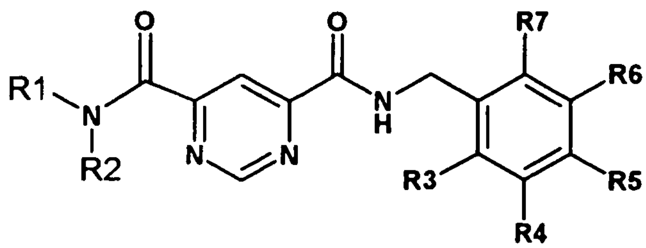

(I)

und/oder alle stereoisomeren Formen der Verbindung der Formel I und/oder Gemische dieser Formen in jedem Verhältnis, und/oder ein physiologisch verträgliches Salz der Verbindung der Formel I, wobei

R1 für Wasserstoffatom oder -($C_1$-$C_6$)-Alkyl steht,
R2 für -($C_1$-$C_6$)-Alkyl steht, wobei Alkyl ein-, zwei- oder dreifach substituiert ist durch -($C_6$-$C_{14}$)-Aryl, worin Aryl ein-, zwei- oder dreifach unabhängig voneinander substituiert ist durch

1) -($C_0$-$C_6$)-Alkyl-C(O)-N(R9)-(R10), worin R9 und R10 gleich oder verschieden sind und unabhängig voneinander

i) Wasserstoffatom oder
ii) -($C_1$-$C_6$)-Alkyl bedeuten oder

R9 und R10 zusammen mit dem Stickstoffatom, an das sie gebunden sind, einen 5-, 6- oder 7-gliedrigen gesättigten Ring bilden, wobei für ein oder zwei weitere Kohlenstoffatome auch ein Heteroatom aus der Reihe Sauerstoff, Schwefel und Stickstoff stehen kann und im Falle von Stickstoff, die Stickstoffatome unabhängig voneinander unsubstituiert oder durch ($C_1$-$C_6$)-Alkyl substituiert sein können,

2) -($C_0$-$C_6$)-Alkyl-C(O)-NH-CN,
3) -O-($C_0$-$C_6$)-Alkyl-C(O)-N(R9)-(R10), worin R9 und R10 die oben genannte Bedeutung haben,
4) -($C_0$-$C_6$)-Alkyl-C(O)-N(R8)-($C_0$-$C_6$)-Alkyl-N(R9)-(R10), worin R8

i) Wasserstoffatom

ii) -($C_1$-$C_6$)-Alkyl, worin Alkyl unsubstituiert ist oder ein- zwei- oder dreifach unabhängig voneinander substituiert ist durch -$NH_2$, -CN, -OH, -C(O)-OH, -C(O)-NH-OH, $NO_2$, -C(O)-O-($C_1$-$C_6$)-Alkyl oder Halogen oder
iii) -OH bedeutet, und
worin R9 und R10 die oben genannte Bedeutung haben,

5) -($C_0$-$C_6$)-Alkyl-C(O)-N(R8)-($C_0$-$C_6$)-Alkyl-Het, wobei R8 die oben genannte Bedeutung hat und Het für ein gesättigtes oder ungesättigtes monocyclisches oder bicyclisches, 3- bis 10-gliedriges heterocyclisches Ringsystem steht, das 1, 2 oder 3 identische oder verschiedene Ringheteroatome aus der Reihe Stickstoff, Sauerstoff und Schwefel enthält und unsubstituiert oder ein- zwei- oder dreifach unabhängig voneinander substituiert ist durch

a) Halogen,
b) Cyano,
c) N itro,
d) Hydroxy,
e) Amino,
f) -C(O)-O-($C_1$-$C_6$)-Alkyl,
g) -C(O)-OH,
h) -($C_1$-$C_6$)-Alkyl, wobei Alkyl unsubstituiert oder ein-, zwei- oder dreifach substituiert ist durch Halogen,
i) -O-($C_1$-$C_6$)-Alkyl, wobei Alkyl unsubstituiert oder ein-, zwei- oder dreifach substituiert ist durch Halogen oder -N(R9)-(R10),
j) =O,
k) -Het,
l) -($C_2$-$C_6$)-Alkenyl, wobei Alkenyl unsubstituiert oder ein-, zwei- oder dreifach substituiert ist durch Halogen oder -N(R9)-(R10), oder
m) -($C_2$-$C_6$)-Alkinyl, wobei Alkinyl unsubstituiert oder ein-, zwei- oder dreifach substituiert ist durch Halogen oder -N(R9)-(R10), oder

6) -($C_0$-$C_6$)-Alkyl-C(O)-N(R8)-($C_0$-$C_6$)-Alkyl-($C_6$-$C_{14}$)-Aryl, wobei Aryl unsubstituiert oder ein- zwei- oder dreifach unabhängig voneinander substituiert ist durch die oben genannten Reste a) bis m),

R3, R4, R5, R6 und R7 gleich oder verschieden sind und unabhängig voneinander für

1. Wasserstoffatom,
2. Halogen,
3. -($C_1$-$C_6$)-Alkyl, worin Alkyl unsubstituiert oder ein-, zwei- oder dreifach durch Halogen substituiert ist,
4. -O-($C_1$-$C_6$)-Alkyl, worin Alkyl unsubstituiert oder ein-, zwei- oder dreifach durch Halogen substituiert ist, oder
5. -S-($C_1$-$C_6$)-Alkyl oder

R4 und R5 oder R5 und R6 zusammen mit den Kohlenstoffatomen, an die sie jeweils gebunden sind unabhängig voneinander einen 5- oder 6-gliedrigen Ring bilden, der aromatisch oder gesättigt ist und Null, ein oder zwei Heteroatome aus der Reihe Sauerstoff, Stickstoff oder Schwefel enthält, wobei der Ring unsubstituiert oder an einem oder an mehreren Kohlenstoffatomen ein- oder zweifach durch Halogen substituiert ist und die anderen Reste R3, R6, und R7 oder R3, R4 und R7 die oben genannte Bedeutung von 1. bis 5. haben.

**2.** Verbindung der Formel I gemäß Anspruch 1, wobei

R1 für Wasserstoffatom oder -($C_1$-$C_6$)-Alkyl steht,
R2 für -($C_1$-$C_6$)-Alkyl steht, wobei Alkyl ein-, zwei- oder dreifach substituiert ist durch Phenyl, worin Phenyl ein-, zwei- oder dreifach unabhängig voneinander substituiert ist durch

1) -($C_0$-$C_6$)-Alkyl-C(O)-N(R9)-(R10), worin R9 und R10 gleich oder verschieden sind und unabhängig voneinander

i) Wasserstoffatom oder
ii) -($C_1$-$C_6$)-Alkyl bedeuten oder

R9 und R10 zusammen mit dem Stickstoffatom, an das sie gebunden sind, einen 5-, 6- oder 7-gliedrigen gesättigten Ring bilden, wobei für ein oder zwei weitere Kohlenstoffatome auch ein Heteroatom aus der Reihe Sauerstoff, Schwefel und Stickstoff stehen kann und im Falle von Stickstoff, die Stickstoffatome unabhängig voneinander unsubstituiert oder durch ($C_1$-$C_6$)-Alkyl substituiert sein können,

2) -($C_0$-$C_6$)-Alkyl-C(O)-NH-CN,
3) -O-($C_0$-$C_6$)-Alkyl-C(O)-N(R9)-(R10), worin R9 und R10 die oben genannte Bedeutung haben,
4) -($C_0$-$C_6$)-Alkyl-C(O)-N(R8)-($C_0$-$C_6$)-Alkyl-N(R9)-(R10), worin R8

i) Wasserstoffatom,
ii) -($C_1$-$C_6$)-Alkyl, worin Alkyl unsubstituiert ist oder ein- zwei- oder dreifach unabhängig voneinander substituiert ist durch -$NH_2$, -CN, -OH, -C(O)-OH, -C(O)-NH-OH, $NO_2$, -C(O)-O-($C_1$-$C_6$)-Alkyl oder Halogen oder
iii) -OH bedeutet, und

worin R9 und R10 die oben genannte Bedeutung haben,
5) -($C_0$-$C_6$)-Alkyl-C(O)-N(R8)-($C_0$-$C_6$)-Alkyl-Het, wobei R8 die oben genannte Bedeutung hat und Het für einen Rest aus der Gruppe Azepin, Azetidin, Aziridin, Benzimidazol, Benzofuran, Benzo[1,4]dioxin, 1,3-Benzodioxol, 4H-Benzo[1,4]oxazin, Benzoxazol, Benzothiazol, Benzothiophen, Chinazolin, Chinolin, Chinoxalin, Chroman, Cinnolin, 1,2-Diazepin, 1,3-Diazepin, 1,4-Diazepin, 1,4-Dioxin, Dioxol, Furan, Imidazol, Indazol, Indol, Isochinolin, Isochroman, Isoindol, Isothiazol, Isoxazol, Morpholin, 1,2-Oxazin, 1,3-Oxazin, 1,4-Oxazin, Oxazol, Oxiran, Piperazin, Piperidin, Phthalazin, Pyran, Pyrazin, Pyrazol, Pyridazin, Pyridin, Pyrimidin, Pyridoimidazol, Pyridopyridin, Pyridopyrimidin, Pyrrol, Pyrrolidin, Tetrazol, 1,2-Thiazin, 1,3-Thiazin, 1,4-Thiazin, Thiazol, Thiomorpholin, Thiophen, Thiopyran, 1,2,3-Triazin, 1,3,5-Triazin, 1,2,4-Triazin, 1,2,3-Triazol oder 1,2,4-Triazol steht und worin Het unsubstituiert ist oder ein- zwei- oder dreifach unabhängig voneinander substituiert ist durch

a) Halogen,
b) Cyano,
c) Nitro,
d) Hydroxy,
e) Amino,
f) -C(O)-O-($C_1$-$C_6$)-Alkyl,
g) -C(O)-OH,
h) -($C_1$-$C_6$)-Alkyl, wobei Alkyl unsubstituiert oder ein-, zwei- oder dreifach substituiert ist durch Halogen,
i) -O-($C_1$-$C_6$)-Alkyl, wobei Alkyl unsubstituiert oder ein-, zwei- oder dreifach substituiert ist durch Halogen oder -N(R9)-(R10),
j) =O,
k) -Het, worin Het wie oben definiert ist,
l) -($C_2$-$C_6$)-Alkenyl, wobei Alkenyl unsubstituiert oder ein-, zwei- oder dreifach substituiert ist durch Halogen oder -N(R9)-(R10), oder
m) -($C_2$-$C_6$)-Alkinyl, wobei Alkinyl unsubstituiert oder ein-, zwei- oder dreifach substituiert ist durch Halogen oder -N(R9)-(R10), oder

6) -($C_0$-$C_6$)-Alkyl-C(O)-N(R8)-($C_0$-$C_6$)-Alkyl-($C_6$-$C_{14}$)-Phenyl, wobei Phenyl unsubstituiert oder ein- zwei- oder dreifach unabhängig voneinander substituiert ist durch die oben genannten Reste a) bis m),

R3, R4, R5, R6 und R7 gleich oder verschieden sind und unabhängig voneinander für

1. Wasserstoffatom,
2. Halogen,
3. -($C_1$-$C_6$)-Alkyl, worin Alkyl unsubstituiert oder ein-, zwei- oder dreifach durch Halogen substituiert ist, oder
4. -O-($C_1$-$C_6$)-Alkyl, worin Alkyl unsubstituiert oder ein-, zwei- oder dreifach durch Halogen substituiert ist,
stehen, oder

R4 und R5 oder R5 und R6 zusammen mit den Kohlenstoffatomen, an die sie jeweils gebunden sind unabhängig voneinander einen Dioxan-, Dioxol-, Dihydrofuran- oder Furan-Ring bilden, wobei der Ring unsubstituiert oder

an einem oder an mehreren Kohlenstoffatomen ein- oder zweifach durch Halogen substituiert ist und die anderen Reste R3, R6, und R7 oder R3, R4 und R7 die oben genannte Bedeutung von 1. bis 4. haben.

**3.** Verbindung der Formel I gemäß der Ansprüche 1 oder 2, wobei

R1 für Wasserstoffatom steht,
R2 für -($C_1$-$C_3$)-Alkyl steht, wobei Alkyl substituiert ist durch Phenyl, worin Phenyl ein-, zwei- oder dreifach unabhängig voneinander substituiert ist durch

1) -($C_0$-$C_6$)-Alkyl-C(O)-N(R9)-(R10), worin R9 und R10 für Wasserstoffatom, Methyl, Ethyl, Propyl oder Butyl steht, oder

R9 und R10 zusammen mit dem Stickstoffatom, an das sie gebunden sind einen Rest bilden, der sich von Pyrrolidin, Piperidin, Pyrazolidin, Pyrazin, Tetrazin, Imidazolidin, Piperazin, Isoxazolidin, Morpholin, Isothiazolidin oder Thiomorpholin ableiten lässt und im Falle von Stickstoff, die Stickstoffatome unabhängig voneinander unsubstituiert oder durch ($C_1$-$C_4$)-Alkyl substituiert sein können,

2) -($C_0$-$C_4$)-Alkyl-C(O)-NH-CN,
3) -O-($C_0$-$C_6$)-Alkyl-C(O)-N(R9)-(R10), worin R9 und R10 die oben genannte Bedeutung haben,
4) -($C_0$-$C_6$)-Alkyl-C(O)-N(R8)-($C_0$-$C_6$)-Alkyl-N(R9)-(R10), worin R8, für Wasserstoff, Methyl, Ethyl, Propyl oder Butyl steht, und R9 und R10 die oben genannte Bedeutung haben,
5) -C(O)-N(R8)-($C_0$-$C_2$)-Alkyl-Het, wobei R8 die oben genannte Bedeutung hat und Het für Azepin, Azetidin, Aziridin, Benzimidazol, Benzofuran, Benzo[1,4]dioxin, 1,3-Benzodioxol, 4H-Benzo[1,4]oxazin, Benzoxazol, Benzothiazol, Benzothiophen, Chinazolin, Chinolin, Chinoxalin, Chroman, Cinnolin, 1,2-Diazepin, 1,3-Diazepin, 1,4-Diazepin, 1,4-Dioxin, Dioxol, Furan, Imidazol, Indazol, Indol, Isochinolin, Isochroman, Isoindol, Isothiazol, Isoxazol, Morpholin, 1,2-Oxazin, 1,3-Oxazin, 1,4-Oxazin, Oxazol, Oxiran, Piperazin, Piperidin, Phthalazin, Pyran, Pyrazin, Pyrazol, Pyridazin, Pyridin, Pyrimidin, Pyridoimidazol, Pyridopyridin, Pyridopyrimidin, Pyrrol, Pyrrolidin, Tetrazol, 1,2-Thiazin, 1,3-Thiazin, 1,4-Thiazin, Thiazol, Thiomorpholin, Thiophen, Thiopyran, 1,2,3-Triazin, 1,3,5-Triazin, 1,2,4-Triazin, 1,2,3-Triazol oder 1,2,4-Triazol steht und Het unsubstituiert oder ein- zwei- oder dreifach unabhängig voneinander substituiert ist durch

a) Halogen,
b) Cyano,
c) Nitro,
d) Hydroxy,
e) Amino,
f) -C(O)-O-($C_1$-$C_4$)-Alkyl,
g) -C(O)-OH,
h) -($C_1$-$C_4$)-Alkyl, wobei Alkyl unsubstituiert oder ein-, zwei- oder dreifach substituiert ist durch Halogen,
i) -O-($C_1$-$C_4$)-Alkyl, wobei Alkyl unsubstituiert oder ein-, zwei- oder dreifach substituiert ist durch Halogen, oder

6) -C(O)-N(R8)-($C_0$-$C_4$)-Alkyl-Phenyl, wobei Phenyl unsubstituiert oder ein- zwei- oder dreifach unabhängig voneinander substituiert ist durch die oben genannten Reste a) bis i),

R3, R4, R5, R6 und R7 gleich oder verschieden sind und unabhängig voneinander für

1. Wasserstoffatom,
2. Halogen,
3. -($C_1$-$C_6$)-Alkyl, worin Alkyl unsubstituiert oder ein-, zwei- oder dreifach durch Halogen substituiert ist,
4. -O-($C_1$-$C_6$)-Alkyl, stehen, worin Alkyl unsubstituiert oder ein-, zwei- oder dreifach durch Halogen substituiert ist, oder

R4 und R5 oder R5 und R6 zusammen mit den Kohlenstoffatomen, an die sie gebunden sind unabhängig voneinander einen Dioxan-, Dioxol-, Dihydrofuran- oder Furan-Ring bilden und die anderen Reste R3, R6, und R7 oder R3, R4 und R7 die oben genannte Bedeutung von 1. bis 4. haben.

**4.** Verbindung der Formel I gemäß einem oder mehreren der Ansprüche 1 bis 3, wobei eine der folgenden Verbindungen

ausgewählt wird.

Pyrimidin-4,6-dicarbonsäure 4-(4-diethylcarbamoyl-benzylamid) 6-(3-methoxybenzylamid),
Pyrimidin-4,6-dicarbonsäure 4-(3-methoxy-benzylamid) 6-(4-propylcarbamoyl-benzylamid),
Pyrimidin-4,6-dicarbonsäure 4-(4-isopropylcarbamoyl-benzylamid) 6-(3-methoxy-benzylamid),
Pyrimidin-4,6-dicarbonsäure 4-[4-(2-dimethylamino-ethylcarbamoyl)-benzylamid] 6-(3-methoxy-benzylamid),
Pyrimidin-4,6-dicarbonsäure 4-[(2,3-dihydro-benz[1,4]dioxin-6-ylmethyl)-amid] 6-[4-(2-dimethylamino-ethylcarbamoyl)-benzylamid],
Pyrimidin-4,6-dicarbonsäure 4-(3-chlor-4-fluor-benzylamid) 6-[4-(2-dimethylamino-ethylcarbamoyl)-benzylamid],
Pyrimidin-4,6-dicarbonsäure4-(4-fluor-3-methyl-benzylamid)6-{4-[2-(4-methylpiperazin-1-yl)-2-oxo-ethyl]-benzylamid},
Pyrimidin-4,6-dicarbonsäure 4-(4-fluor-3-methyl-benzylamid) 6-[4-(2-morpholin-4-yl-2-oxo-ethoxy)-benzylamid],
Pyrimidin-4,6-dicarbonsäure 4-(4-diethylcarbamoylmethoxy-benzylamid) 6-(4-fluor-3-methyl-benzylamid),
Pyrimidin-4,6-dicarbonsäure 4-(4-fluor-3-methyl-benzylamid) 6-[4-(isopropylcarbamoyl-methyl)-benzylamid],
Pyrimidin-4,6-dicarbonsäure 4-(4-fluor-3-methyl-benzylamid)6-{4-[(2-morpholin-4-yl-ethylcarbamoyl)-methyl]-benzylamid},
Pyrimidin-4,6-dicarbonsäure 4-(4-diethylcarbamoylmethyl-benzylamid)6-(4-fluor-3-methyl-benzylamid),
Pyrimidin-4,6-dicarbonsäure 4-(4-fluor-3-methyl-benzylamid) 6-[4-(2-morpholin-4-yl-2-oxo-ethyl)-benzylamid],
Pyrimidin-4,6-dicarbonsäure 4-(4-fluor-3-methyl-benzylamid) 6-[4-(isopropylcarbamoyl-methoxy)-benzylamid],
Pyrimidin-4,6-dicarbonsäure 4-(3-methoxy-benzylamid)6-[4-(2-morpholin-4-yl-ethylcarbamoyl)-benzylamid],
Pyrimidin-4,6-dicarbonsäure 4-(3-methoxy-benzylamid)6-[4-(2-pyrrolidin-1-yl-ethylcarbamoyl)-benzylamid],
Pyrimidin-4,6-dicarbonsäure 4-(4-fluor-3-methyl-benzylamid) 6-[4-(thiomorpholin-4-carbonyl)-benzylamid],
Pyrimidin-4,6-dicarbonsäure 4-(3-methoxy-benzylamid)6-[4-(thiomorpholin-4-carbonyl)-benzylamid],
Pyrimidin-4,6-dicarbonsäure 4-(4-cyancarbamoyl-benzylamid)6-(4-fluor-3-methyl-benzylamid),
Pyrimidin-4,6-dicarbonsäure 4-(3-methoxy-benzylamid) 6-[4-(3-morpholin-4-yl-propylcarbamoyl)-benzylamid],
Pyrimidin-4,6-dicarbonsäure 4-[(2,3-dihydro-benzofuran-5-ylmethyl)-amid] 6-[4-(3-morpholin-4-yl-propylcarbamoyl)-benzylamid],
Pyrimidin-4,6-dicarbonsäure 4-(4-fluor-3-methyl-benzylamid) 6-[4-(4-methylpiperazin-1-carbonyl)-benzylamid],
Pyrimidin-4,6-dicarbonsäure 4-[(2,3-dihydro-benzofuran-5-ylmethyl)-amid] 6-{4-[(pyridin-4-ylmethyl)-carbamoyl]-benzylamid},
Pyrimidin-4,6-dicarbonsäure 4-(4-N-cyan-carbamoyl-benzylamid)6-[(2,3-dihydro-benzfuran-5-ylmethyl)-amid],
Pyrimidin-4,6-dicarbonsäure 4-(4-N-cyan-carbamoyl-benzylamid)6-(3-methoxy-benzylamid),
Pyrimidin-4,6-dicarbonsäure 4-(4-fluor-3-methyl-benzylamid) 6-[4-(morpholin-4-carbonyl)-benzylamid],
Pyrimidin-4,6-dicarbonsäure 4-(4-fluor-3-methyl-benzylamid) 6-[4-(2-piperazin-1-yl-ethylcarbamoyl)-benzylamid],
Pyrimidin-4,6-dicarbonsäure 4-(4-tert-butylcarbamoyl-benzylamid)6-(3-methoxy-benzylamid),
Pyrimidin-4,6-dicarbonsäure 4-(3-methoxy-benzylamid)6-{4-[methyl-(1-methylpiperidin-4-yl)-carbamoyl]-benzylamid},
Pyrimidin-4,6-dicarbonsäure 4-(4-fluor-3-methyl-benzylamid)6-[4-(2-pyrrolidin-1-yl-ethylcarbamoyl)-benzylamid],
Pyrimidin-4,6-dicarbonsäure 4-(3-methoxy-benzylamid) 6-[3-(2-morpholin-4-yl-ethylcarbamoyl)-benzylamid],
Pyrimidin-4,6-dicarbonsäure 4-(4-fluor-3-methyl-benzylamid) 6-[4-(2-morpholin-4-yl-ethylcarbamoyl)-benzylamid],
[4-({[6-(4-Fluor-3-methyl-benzylcarbamoyl)-pyrimidin-4-carbonyl]-amino}-methyl)-benzoylamino]-acetic acid methyl ester,
Pyrimidin-4,6-dicarbonsäure 4-(3-methoxy-benzylamid) 6-[3-(morpholin-4-carbonyl)-benzylamid],
Pyrimidin-4,6-dicarbonsäure 4-(3-methoxy-benzylamid) 6-{4-[(piperidin-4-ylmethyl)-carbamoyl]-benzylamid},
Pyrimidin-4,6-dicarbonsäure 4-(3-methoxy-benzylamid) 6-[4-(piperidin-4-ylcarbamoyl)-benzylamid],
Pyrimidin-4,6-dicarbonsäure 4-(4-fluor-3-methyl-benzylamid) 6-[4-(piperidin-4-ylcarbamoyl)-benzylamid],
Pyrimidin-4,6-dicarbonsäure 4-(4-fluor-3-methyl-benzylamid) 6-{4-[methyl-(1-methyl-piperidin-4-yl)-carbamoyl]-benzylamid},
Pyrimidin-4,6-dicarbonsäure 4-(4-fluor-3-methyl-benzylamid)6-{4-[(piperidin-4-ylmethyl)-carbamoyl]-benzylamid},
Pyrimidin-4,6-dicarbonsäure4-(3-methoxy-benzylamid)6-[4-(4-methylpiperazin-1-carbonyl)-benzylamid],

Pyrimidin-4,6-dicarbonsäure 4-[(2,3-dihydro-benzofuran-5-ylmethyl)-amid]6-[4-(morpholin-4-carbonyl)-benzylamid],
Pyrimidin-4,6-dicarbonsäure 4-[(2,3-dihydro-benzofuran-5-ylmethyl)-amid]6-[4-(4-methyl-piperazin-1-carbonyl)-benzylamid],
Pyrimidin-4,6-dicarbonsäure 4-[(2,3-dihydro-benzfuran-5-ylmethyl)-amid]6-[4-(2-pyrrolidin-1-yl-ethylcarbamoyl)-benzylamid],
Pyrimidin-4,6-dicarbonsäure 4-[(2,3-dihydro-benzofuran-5-ylmethyl)-amid]6-[4-(2-morpholin-4-yl-ethylcarbamoyl)-benzylamid],
Pyrimidin-4,6-dicarbonsäure 4-(3-chlor-4-fluor-benzylamid)6-[4-(2-pyrrolidin-1-yl-ethylcarbamoyl)-benzylamid],
Pyrimidin-4,6-dicarbonsäure 4-(3-methoxy-benzylamid)6-[4-(morpholin-4-carbonyl)-benzylamid],
Pyrimidin-4,6-dicarbonsäure 4-(3-methoxy-benzylamid)6-{4-[(pyridin-4-ylmethyl)-carbamoyl]-benzylamid},
Pyrimidin-4,6-dicarbonsäure 4-(3-chlor-4-fluor-benzylamid)6-(4-diethylcarbamoyl-benzylamid),
Pyrimidin-4,6-dicarbonsäure 4-(3-chlor-4-fluor-benzylamid)6-[4-(morpholin-4-carbonyl)-benzylamid],
Pyrimidin-4,6-dicarbonsäure 4-(3-chlor-4-fluor-benzylamid)6-[4-(2-morpholin-4-yl-ethylcarbamoyl)-benzylamid] oder
Pyrimidin-4,6-dicarbonsäure 4-(4-diethylcarbamoyl-benzylamid)6-(3-methoxybenzylamid).

5. Verfahren zur Herstellung der Verbindung der Formel I gemäß einem oder mehreren der Ansprüche 1 bis 4, **dadurch gekennzeichnet, dass** man eine Verbindung der Formel II

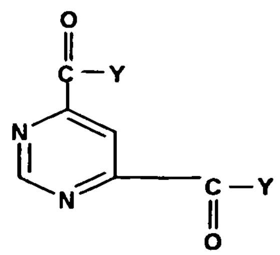

( II )

a) mit einer Verbindung der Formel IIIa oder IIIb

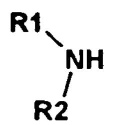

IIIa

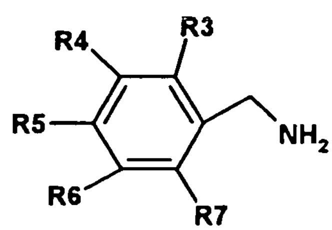

IIIb

umsetzt, wobei R1, R2, R3, R4, R5, R6 und R7 die in Formel I angegebenen Bedeutungen haben und Y Halogen, Hydroxyl oder $C_1$-$C_4$-Alkoxy ist oder zusammen mit der Carbonylgruppe einen Aktivester oder ein gemischtes Anhydrid bildet, wobei eine Verbindung der Formel I gebildet wird und die Reaktionsprodukte gegebenenfalls in ihre physiologisch verträglichen Salze überführt, oder
b) eine Verbindung der Formel II mit einer Verbindung der Formel IIIa oder IIIb zu einer Verbindung der Formel IVa oder IVb.

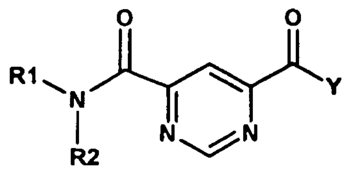

(IVa)

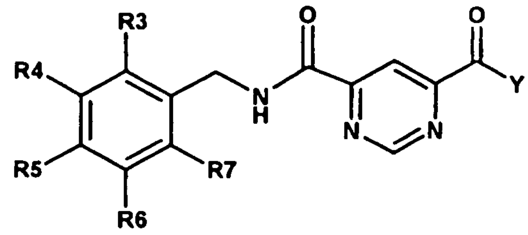

(IVb)

umsetzt, wobei R1 bis R7 die in Formel I angegebenen Bedeutungen haben und Y Halogen, Hydroxyl oder $C_1$-$C_4$-Alkoxy ist oder zusammen mit der Carbonylgruppe einen Aktivester oder ein gemischtes Anhydrid bildet und die Verbindung der Formel IVa oder IVb gegebenenfalls reinigt und anschließend mit einer Verbindung der Formel IIIa oder IIIb in eine Verbindung der Formel I überführt.

**6.** Arzneimittel, **gekennzeichnet durch** einen wirksamen Gehalt von mindestens einer Verbindung der Formel I gemäß einem oder mehreren der Ansprüche 1 bis 4 zusammen mit einem pharmazeutisch geeigneten und physiologisch verträglichen Trägerstoff, Zusatzstoff und/oder anderen Wirk- und Hilfsstoffen.

**7.** Verwendung der Verbindung der Formel I gemäß einem oder mehreren der Ansprüche 1 bis 4 zur Herstellung eines Arzneimittels zur Prophylaxe und Therapie degenerativen Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Ferner gehören dazu auch Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels oder Krebserkrankungen wie Brustkrebs handelt.

**Claims**

**1.** A compound of the formula I

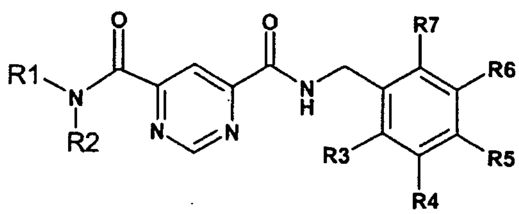

(I)

and/or all the stereoisomeric forms of the compound of the formula I and/or mixtures of these forms in any ratio, and/or a physiologically tolerated salt of the compound of the formula I, wherein

R1 is hydrogen atom or -($C_1$-$C_6$)-alkyl,
R2 is -($C_1$-$C_6$)-alkyl, where alkyl is substituted, once, twice or three times, by -($C_6$-$C_{14}$)-aryl, in which aryl is substituted, once, twice or three times, independently of each other, by

1) -($C_0$-$C_6$)-alkyl-C(O)-N(R9)-(R10), in which R9 and R10 are identical or different and are, independently of each other,

i) hydrogen atom or
ii) -($C_1$-$C_6$)-alkyl, or
R9 and R10 form, together with the nitrogen atom to which they are bonded, a 5-, 6- or 7-membered saturated ring, where a heteroatom from the series oxygen, sulfur and nitrogen can also replace one

or two further carbon

atoms and, in the case of nitrogen, the nitrogen atoms can, independently of each other, be unsubstituted or substituted by $(C_1-C_6)$-alkyl,
2) $-(C_0-C_6)$-alkyl-C(O)-NH-CN,
3) -O-$(C_0-C_6)$-alkyl-C(O)-N(R9)-(R10), in which R9 and R10 have the abovementioned meaning,
4) $-(C_0-C_6)$-alkyl-C(O)-N(R8)-$(C_0-C_6)$-alkyl-N(R9)-(R10), in which R8 is

i) hydrogen atom
ii) $-(C_1-C_6)$-alkyl, in which alkyl is unsubstituted or substituted, once, twice or three times, independently of each other, by $-NH_2$, -CN, -OH, -C (O) -OH, -C (O) -NH-OH, $NO_2$, -C(O)-O-$(C_1-C_6)$-alkyl, or halogen, or
iii) OH, and

in which R9 and R10 have the abovementioned meaning,
5) $-(C_0-C_6)$-alkyl-C(O)-N(R8)-$(C_0-C_6)$-alkyl-Het, where R8 has the abovementioned meaning and Het is a saturated or unsaturated, monocyclic or bicyclic, 3- to 10-membered heterocyclic ring system which contains 1, 2 or 3 identical or different ring heteroatoms from the series nitrogen, oxygen and sulfur and is unsubstituted or substituted, once, twice or three times, independently of each other, by

a) halogen,
b) cyano,
c) nitro,
d) hydroxyl,
e) amino,
f) -C(O)-O-$(C_1-C_6)$-alkyl,
g) -C(O)-OH,
h) $-(C_1-C_6)$-alkyl, where alkyl is unsubstituted or substituted, once, twice or three times, by halogen,
i) -O- $(C_1-C_6)$ -alkyl, where alkyl is unsubstituted or substituted, once, twice or three times, by halogen, or -N(R9)-(R10),
j) =O,
k) -Het,
l) $-(C_2-C_6)$-alkenyl, where alkenyl is unsubstituted or substituted, once, twice or three times, by halogen, or -N(R9)-(R10), or
m) $-(C_2-C_6)$-alkynyl, where alkynyl is unsubstituted or substituted, once, twice or three times, by halogen or -N(R9)-(R10), or

6) $-(C_0-C_6)$-alkyl-C(O)-N(R8)-$(C_0-C_6)$-alkyl-$(C_6-C_{14})$ -aryl, where aryl is unsubstituted or substituted, once, twice or three times, independently of each other, by the abovementioned radicals a) to m),

R3, R4, R5, R6 and R7 are identical or different and are, independently of each other,

1. hydrogen atom,
2. halogen,
3. $-(C_1-C_6)$-alkyl in which alkyl is unsubstituted or substituted, once, twice or three times, by halogen,
4. -O-$(C_1-C_6)$-alkyl in which alkyl is unsubstituted or substituted, once, twice or three times, by halogen, or
5. -S-$(C_1-C_6)$-alkyl, or

R4 and R5 or R5 and R6 form, together with the carbon atoms to which they are in each case bonded, independently of each other, a 5- or 6-membered ring which is aromatic or saturated and contains zero, one or two heteroatoms from the series oxygen, nitrogen or sulfur, where the ring is unsubstituted or is substituted, at one or at several carbon atoms, once or twice, by halogen and the other radicals R3, R6 and R7 or R3, R4 and R7 have the abovementioned meaning of 1. to 5.

**2.** A compound of the formula I as claimed in claim 1, wherein

R1 is hydrogen atom or $-(C_1-C_6)$-alkyl,
R2 is $-(C_1-C_6)$-alkyl, where alkyl is substituted, once, twice or three times, by phenyl, in which phenyl is substituted, once, twice or three times, independently of each other, by

1) -($C_0$-$C_6$)-alkyl-C(O)-N(R9)-(R10), in which R9 and R10 are identical or different and are, independently of each other,

i) hydrogen atom or
ii) -($C_1$-$C_6$)-alkyl, or
R9 and R10 form, together with the nitrogen atom to which they are bonded, a 5-, 6- or 7-membered saturated ring, where a heteroatom from the series oxygen, sulfur and nitrogen can also replace one or two further carbon atoms and, in the case of nitrogen, the nitrogen atoms can, independently of each other, be unsubstituted or substituted by ($C_1$-$C_6$)-alkyl,

2) -($C_0$-$C_6$)-alkyl-C(O)-NH-CN,
3) -O-($C_0$-$C_6$)-alkyl-C(O)-N(R9)-(R10), in which R9 and R10 have the abovementioned meaning,
4) -($C_0$-$C_6$)-alkyl-C(O)-N(R8)-alkyl-N(R9)-(R10), in which R8 is

i) hydrogen atom
ii) -($C_1$-$C_6$)-alkyl, in which alkyl is unsubstituted or substituted, once, twice or three times, independently of each other, by -$NH_2$, -CN, -OH, -C(O)-OH, -C (O) -NH-OH, $NO_2$, -C(O)-O-($C_1$-$C_6$)-alkyl, or halogen or
iii) -OH, and in which R9 and R10 have the abovementioned meaning,

5) -($C_0$-$C_6$)-alkyl-C(O)-N(R8)-($C_0$-$C_6$)-alkyl-Het, where R8 has the abovementioned meaning and Het is a radical from the group azepine, azetidine, aziridine, benzimidazole, benzofuran, benzo[1,4]dioxin, 1,3-benzodioxole, 4H-benzo[1,4]oxazine, benzoxazole, benzothiazole, benzothiophene, quinazoline, quinoline, quinoxaline, chroman, cinnoline, 1,2-diazepine, 1,3-diazepine, 1,4-diazepine, 1,4-dioxin, dioxole, furan, imidazole, indazole, indole, isoquinoline, isochroman, isoindole, isothiazole, isoxazole, morpholine, 1,2-oxazine, 1,3-oxazine, 1,4-oxazine, oxazole, oxiran, piperazine, piperidine, phthalazine, pyran, pyrazine, pyrazole, pyridazine, pyridine, pyrimidine, pyridoimidazole, pyridopyridine, pyridopyrimidine, pyrrole, pyrrolidine, tetrazole, 1,2-thiazine, 1,3-thiazine, 1,4-thiazine, thiazole, thiomorpholine, thiophene, thiopyran, 1,2,3-triazine, 1,3,5-triazine, 1,2,4-triazine, 1,2,3-triazole or 1,2,4-triazole, and in which Het is unsubstituted or substituted, once, twice or three times, independently of each other, by

a) halogen,
b) cyano,
c) nitro,
d) hydroxyl,
e) amino,
f) -C(O)-O-($C_1$-$C_6$)-alkyl,
g) -C(O)-OH,
h) -($C_1$-$C_6$)-alkyl, where alkyl is unsubstituted or substituted, once, twice or three times, by halogen,
i) -O-($C_1$-$C_6$)-alkyl, where alkyl is unsubstituted or substituted, once, twice or three times, by halogen, or -N(R9)-(R10),
j) =O,
k) -Het, in which Het is defined as above,
l) -($C_2$-$C_6$)-alkenyl, where alkenyl is unsubstituted or substituted, once, twice or three times, by halogen, or -N(R9)-(R10), or
m) -($C_2$-$C_6$)-alkynyl, where alkynyl is unsubstituted or substituted, once, twice or three times, by halogen or -N(R9)-(R10), or

6) -($C_0$-$C_6$)-alkyl-C(O)-N(R8)-($C_0$-$C_6$)-alkyl-($C_6$-$C_{14}$)-phenyl, where phenyl is unsubstituted or substituted, once, twice or three times, independently of each other, by the abovementioned radicals a) to m),

R3, R4, R5, R6 and R7 are identical or different and are, independently of each other,

1. hydrogen atom,
2. halogen,
3. -($C_1$-$C_6$)-alkyl, in which alkyl is unsubstituted or substituted, once, twice or three times, by halogen, or
4. -O-($C_1$-$C_6$)-alkyl, in which alkyl is unsubstituted or substituted, once, twice or three times, by halogen, or

R4 and R5 or R5 and R6 form, together with the carbon atoms to which they are in each case bonded, independently

of each other, a dioxane, dioxole, dihydrofuran or furan ring, where the ring is unsubstituted or substituted, at one or at several carbon atoms, once or twice, by halogen and the other radicals R3, R6 and R7 or R3, R4 and R7 have the abovementioned meaning of 1. to 4..

**3.** A compound of the formula I as claimed in claim 1 or 2, wherein

R1 is hydrogen atom,
R2 is -($C_1$-$C_3$)-alkyl, where alkyl is substituted by phenyl, in which phenyl is substituted, once, twice or three times, independently of each other, by

1) -($C_0$-$C_6$)-alkyl-C(O)-N(R9)-(R10), in which R9 and R10 are hydrogen atom, methyl, ethyl, propyl or butyl, or
R9 and R10 form, together with the nitrogen atom to which they are bonded, a radical which can be derived from pyrrolidine, piperidine, pyrazolidine, pyrazine, tetrazine, imidazolidine, piperazine, isoxazolidine, morpholine, isothiazolidine or thiomorpholine, and, in the case of nitrogen, the nitrogen atoms can, independently of each other, be unsubstituted or substituted by ($C_1$-$C_4$)-alkyl,
2) -($C_0$-$C_4$)-alkyl-C(O)-NH-CN,
3) -O-($C_0$-$C_6$)-alkyl-C(O)-N(R9)-(R10), in which R9 and R10 have the abovementioned meaning,
4) -($C_0$-$C_6$)-alkyl-C(O)-N(R8)-($C_0$-$C_6$)-alkyl-N(R9)-(R10), in which R8 is hydrogen, methyl, ethyl, propyl or butyl, and R9 and R10 have the abovementioned meaning,
5) -C(O)-N(R8)-($C_0$-$C_2$)-alkyl-Het, where R8 has the abovementioned meaning and Het is azepine, azetidine, aziridine, benzimidazole, benzofuran, benzo [1,4] dioxin, 1,3-benzodioxole, 4H-benzo [1,4] oxazine, benzoxazole, benzothiazole, benzothiophene, quinazoline, quinoline, quinoxaline, chroman, cinnoline, 1,2-diazepine, 1,3-diazepine, 1,4-diazepine, 1,4-dioxin, dioxole, furan, imidazole, indazole, indole, isoquinoline, isochroman, isoindole, isothiazole, isoxazole, morpholine, 1,2-oxazine, 1,3-oxazine, 1,4-oxazine, oxazole, oxirane, piperazine, piperidine, phthalazine, pyran, pyrazine, pyrazole, pyridazine, pyridine, pyrimidine, pyridoimidazole, pyridopyridine, pyridopyrimidine, pyrrole, pyrrolidine, tetrazole, 1,2-thiazine, 1,3-thiazine, 1,4-thiazine, thiazole, thiomorpholine, thiophene, thiopyran, 1,2,3-triazine, 1,3,5-triazine, 1,2,4-triazine, 1,2,3-triazole or 1,2,4-triazole, and Het is unsubstituted or substituted, once, twice or three times, independently of each other, by

a) halogen
b) cyano,
c) nitro,
d) hydroxyl,
e) amino,
f) -C(O)-O-($C_1$-$C_4$)-alkyl,
g) -C(O)-OH,
h) -($C_1$-$C_4$)-alkyl, where alkyl is unsubstituted or substituted, once, twice or three times, by halogen,
i) -O-($C_1$-$C_4$)-alkyl, where alkyl is unsubstituted or substituted, once, twice or three times, by halogen, or

6) -C(O)-N(R8)-($C_0$-$C_4$)-alkyl-phenyl, where phenyl is unsubstituted or substituted, once, twice or three times, independently of each other, by the abovementioned radicals a) to i),

R3, R4, R5, R6 and R7 are identical or different and are, independently of each other,

1. hydrogen atom,
2. halogen,
3. -($C_1$-$C_6$)-alkyl, in which alkyl is unsubstituted or substituted, once, twice or three times, by halogen,
4. -O-($C_1$-$C_6$)-alkyl, in which alkyl is unsubstituted or substituted, once, twice or three times, by halogen, or

R4 and R5 or R5 and R6 form, together with the carbon atoms to which they are bonded, independently of each other, a dioxane, dioxole, dihydrofuran or furan ring and the other radicals R3, R6 and R7 or R3, R4 and R7 have the abovementioned meaning of 1. to 4..

**4.** A compound of the formula I as claimed in one or more of claims 1 to 3, wherein one of the following compounds is selected

pyrimidine-4,6-dicarboxylic acid 4-(4-diethylcarbamoylbenzylamide) 6-(3-methyloxybenzylamide),
pyrimidine-4,6-dicarboxylic acid 4-(3-methoxybenzylamide) 6-(4-propylcarbamoyl benzylamide),
pyrimidine-4,6-dicarboxylic acid 4-(4-isopropylcarbamoylbenzylamide) 6-(3-methoxybenzylamide),
pyrimidine-4,6-dicarboxylic acid 4-[4-(2-dimethylaminoethylcarbamoyl)benzylamide] 6-(3-methoxybenzylamide),
pyrimidine-4,6-dicarboxylic acid 4-[(2,3-dihydrobenzo[1,4]dioxin-6-ylmethyl)amide] 6-[4-(2-dimethylaminoethylcarbamoyl)benzylamide],
pyrimidine-4,6-dicarboxylic acid 4-(3-chloro-4-fluorobenzylamide) 6-[4-(2-dimethylaminoethylcarbamoyl)benzylamide],
pyrimidine-4,6-dicarboxylic acid 4-(4-fluoro-3-methylbenzylamide) 6-{4-[2-(4-methylpiperazin-1-yl)-2-oxoethyl] benzylamide},
pyrimidine-4,6-dicarboxylic acid 4-(4-fluoro-3-methylbenzylamide) 6-[4-(2-morpholin-4-yl-2-oxoethoxy)benzylamide],
pyrimidine-4,6-dicarboxylic acid 4-(4-diethylcarbamoylmethoxybenzylamide) 6-(4-fluoro-3-methylbenzylamide),
pyrimidine-4,6-dicarboxylic acid 4-(4-fluoro-3-methylbenzylamide) 6-[4-(isopropylcarbamoylmethyl)benzylamide],
pyrimidine-4,6-dicarboxylic acid 4-(4-fluoro-3-methylbenzylamide) 6-{4-[(2-morpholin-4-ylethylcarbamoyl)methyl]benzylamide},
pyrimidine-4,6-dicarboxylic acid 4-(4-diethylcarbamoylmethylbenzylamide) 6-(4-fluoro-3-methylbenzylamide),
pyrimidine-4,6-dicarboxylic acid 4-(4-fluoro-3-methylbenzylamide) 6-[4-(2-morpholin-4-yl-2-oxoethyl)benzylamide],
pyrimidine-4,6-dicarboxylic acid 4-(4-fluoro-3-methylbenzylamide) 6-[4-(isopropylcarbamoylmethoxy)benzylamide],
pyrimidine-4,6-dicarboxylic acid 4-(3-methoxybenzylamide) 6-[4-(2-morpholin-4-ylethylcarbamoyl)benzylamide],
pyrimidine-4,6-dicarboxylic acid 4-(3-methoxybenzylamide) 6-[4-(2-pyrrolidin-1-yl-ethylcarbamoyl)benzylamide],
pyrimidine-4,6-dicarboxylic acid 4-(4-fluoro-3-methylbenzylamide) 6-[4-(thiomorpholine-4-carbonyl)benzylamide],
pyrimidine-4,6-dicarboxylic acid 4-(3-methoxybenzylamide) 6-[4-(thiomorpholine-4-carbonyl)benzylamide],
pyrimidine-4,6-dicarboxylic acid 4-(4-cyanocarbamoylbenzylamide) 6-(4-fluoro-3-methylbenzylamide),
pyrimidine-4,6-dicarboxylic acid 4-(3-methoxybenzylamide) 6-[4-(3-morpholin-4-ylpropylcarbamoyl)benzylamide],
pyrimidine-4,6-dicarboxylic acid 4-[(2,3-dihydrobenzofuran-5-ylmethyl)amide] 6-[4-(3-morpholin-4-yl-propylcarbamoyl)benzylamide],
pyrimidine-4,6-dicarboxylic acid 4-(4-fluoro-3-methylbenzylamide) 6-[4-(4-methylpiperazine-1-carbonyl)benzylamide],
pyrimidine-4,6-dicarboxylic acid 4-[(2,3-dihydrobenzofuran-5-ylmethyl)amide] 6-{4-[(pyridin-4-ylmethyl)carbamoyl]benzylamide},
pyrimidine-4,6-dicarboxylic acid 4-(4-N-cyanocarbamoylbenzylamide) 6-[(2,3-dihydrobenzofuran-5-ylmethyl) amide],
pyrimidine-4,6-dicarboxylic acid 4-(4-N-cyanocarbamoylbenzylamide) 6-(3-methoxybenzylamide),
pyrimidine-4,6-dicarboxylic acid 4-(4-fluoro-3-methylbenzylamide) 6-[4-(morpholine-4-carbonyl)benzylamide],
pyrimidine-4,6-dicarboxylic acid 4-(4-fluoro-3-methylbenzylamide) 6-[4-(2-piperazin-1-ylethylcarbamoyl)benzylamide],
pyrimidine-4,6-dicarboxylic acid 4-(4-tert-butylcarbamoylbenzylamide) 6-(3-methoxybenzylamide),
pyrimidine-4,6-dicarboxylic acid 4-(3-methoxybenzylamide) 6-{4-[methyl-(1-methylpiperidin-4-yl)carbamoyl] benzylamide},
pyrimidine-4,6-dicarboxylic acid 4-(4-fluoro-3-methylbenzylamide) 6-[4-(2-pyrrolidin-1-yl-ethylcarbamoyl)benzylamide],
pyrimidine-4,6-dicarboxylic acid 4-(3-methoxybenzylamide) 6-[3-(2-morpholin-4-ylethylcarbamoyl)benzylamide],
pyrimidine-4,6-dicarboxylic acid 4-(4-fluoro-3-methylbenzylamide) 6-[4-(2-morpholin-4-ylethylcarbamoyl) benzylamide],
[4-({[6-(4-fluoro-3-methylbenzylcarbamoyl)pyrimidine-4-carbonyl]-amino}methyl)benzoylamino]acetic acid methyl ester,
pyrimidine-4,6-dicarboxylic acid 4-(3-methoxybenzylamide) 6-[3-(morpholine-4-carbonyl)benzylamide],

pyrimidine-4,6-dicarboxylic acid 4-(3-methoxybenzylamide) 6-{4-[(piperidin-4-ylmethyl)carbamoyl]benzylamide},
pyrimidine-4,6-dicarboxylic acid 4-(3-methoxybenzylamide) 6-[4-(piperidin-4-ylcarbamoyl)benzylamide],
pyrimidine-4,6-dicarboxylic acid 4-(4-fluoro-3-methylbenzylamide) 6-[4-(piperidin-4-ylcarbamoyl)benzylamide],
pyrimidine-4,6-dicarboxylic acid 4-(4-fluoro-3-methylbenzylamide) 6-{4-[methyl-(1-methylpiperidin-4-yl)carbamoyl]benzylamide},
pyrimidine-4,6-dicarboxylic acid 4-(4-fluoro-3-methylbenzylamide) 6-{4-[(piperidin-4-ylmethyl)carbamoyl]benzylamide},
pyrimidine-4,6-dicarboxylic acid 4-(3-methoxybenzylamide) 6-[4-(4-methyl-piperazine-1-carbonyl)benzylamide],
pyrimidine-4,6-dicarboxylic acid 4-[(2,3-dihydrobenzofuran-5-ylmethyl)amide] 6-[4-(morpholine-4-carbonyl) benzylamide],
pyrimidine-4,6-dicarboxylic acid 4-[(2,3-dihydrobenzofuran-5-ylmethyl)amide] 6-[4-(4-methylpiperazine-1-carbonyl)benzylamide],
pyrimidine-4,6-dicarboxylic acid 4-[(2,3-dihydrobenzofuran-5-ylmethyl)amide] 6-[4-(2-pyrrolidin-1-yl-ethylcarbamoyl)benzylamide],
pyrimidine-4,6-dicarboxylic acid 4-[(2,3-dihydrobenzofuran-5-ylmethyl)amide] 6-[4-(2-morpholin-4-ylethylcarbamoyl)benzylamide],
pyrimidine-4,6-dicarboxylic acid 4-(3-chloro-4-fluorobenzylamide) 6-[4-(2-pyrrolidin-1-ylethylcarbamoyl)benzylamide],
pyrimidine-4,6-dicarboxylic acid 4-(3-methoxybenzylamide) 6-[4-(morpholine-4-carbonyl)benzylamide],
pyrimidine-4,6-dicarboxylic acid 4-(3-methoxybenzylamide) 6-{4-[(pyridin-4-ylmethyl)carbamoyl]benzylamide},
pyrimidine-4,6-dicarboxylic acid 4-(3-chloro-4-fluorobenzylamide) 6-(4-diethylcarbamoylbenzylamide),
pyrimidine-4,6-dicarboxylic acid 4-(3-chloro-4-fluorobenzylamide) 6-[4-(morpholine-4-carbonyl)benzylamide],
pyrimidine-4,6-dicarboxylic acid 4-(3-chloro-4-fluorobenzylamide) 6-[4-(2-morpholin-4-ylethylcarbamoyl)benzylamide], or
pyrimidine-4,6-dicarboxylic acid 4-(4-diethylcarbamoylbenzylamide) 6-(3-methoxybenzylamide).

**5.** A process for preparing the compound of the formula I as claimed in one or more of claims 1 to 4, wherein a compound of the formula II

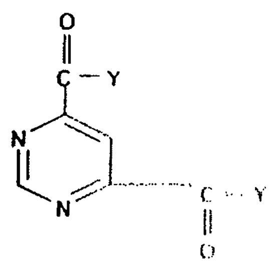

( II )

a) is reacted with a compound of the formula IIIa or IIIb

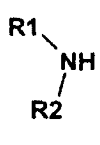

IIIa

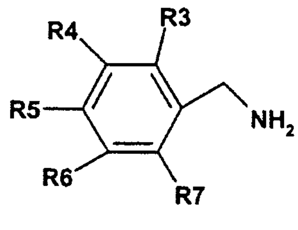

IIIb

where R1, R2, R3, R4, R5, R6 and R7 have the meanings given in formula I and Y is halogen, hydroxyl or $C_1$-$C_4$-alkoxy or forms, together with the carbonyl group, an active ester or a mixed anhydride, with a compound of the formula I being formed, and the reaction products are converted, where appropriate, into their physiologically tolerated salts, or
b) a compound of the formula II is reacted with a compound of the formula IIIa or IIIb to give a compound of

the formula IVa or IVb

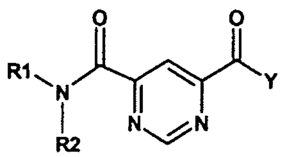

(IVa)

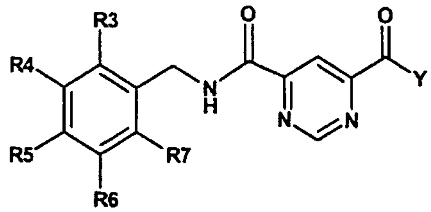

(IVb)

where R1 to R7 have the meanings given in formula I and Y is halogen, hydroxyl or $C_1$-$C_4$-alkoxy, or forms, together with the carbonyl group, an active ester or a mixed anhydride, and the compound of the formula IVa or IVb is purified, where appropriate, and then converted, with a compound of the formula Ilia or IIIb, into a compound of the formula I.

**6.** A pharmaceutical, which comprises an effective content of at least one compound of the formula I as claimed in one or more of claims 1 to 4 together with a pharmaceutically suitable and physiologically tolerated carrier substance, additive and/or other active compounds and auxiliary substances.

**7.** The use of the compound of the formula I as claimed in one or more of claims 1 to 4 for producing a pharmaceutical for the prophylaxis and therapy of degenerative joint diseases such as osteoarthroses, spondyloses, cartilage loss following joint trauma or relatively long joint immobilization following meniscus or patella injuries or ligament rupture, they also include diseases of the connective tissue such as collagenoses, periodontal diseases, wound healing disturbances and chronic diseases of the locomotory apparatus such as inflammatory, immunologically determined or metabolism-determined, acute and chronic arthritides, arthropathies, myalgias and disturbances of bone metabolism or cancer diseases such as breast cancer.

## Revendications

**1.** Composé de formule I

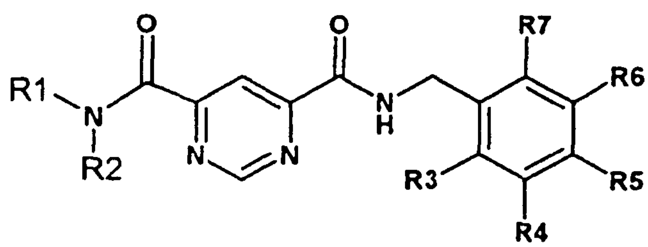

(I)

et/ou toutes les formes stéréo-isomères du composé de formule I et/ou les mélanges de ces formes, dans tous les rapports, et/ou un sel physiologiquement acceptable du composé de formule (I), où

R1 représente un atome d'hydrogène ou -($C_1$-$C_6$)-alkyle,
R2 représente -($C_1$-$C_6$)-alkyle, où alkyle est monosubstitué, disubstitué ou trisubstitué par -($C_6$-$C_{14}$)-aryle, où aryle est monosubstitué, disubstitué ou trisubstitué, indépendamment l'un de l'autre, par

1) -($C_0$-$C_6$)-alkyl-C(O)-N(R9)-(R10), où R9 et R10 sont identiques ou différents et signifient, indépendamment l'un de l'autre

i) un atome d'hydrogène ou
ii) -($C_1$-$C_6$)-alkyle ou

R9 et R10 forment ensemble avec l'atome d'azote auquel ils sont liés, un cycle saturé de 5, 6 ou 7 chaînons, où un ou deux atomes de carbone peuvent également être un hétéroatome de la série oxygène, soufre et azote, et où, dans le cas de l'azote, les atomes d'azote peuvent être non substitués ou être substitués, indépendamment les uns des autres, par ($C_1$-$C_6$) -alkyle,

2) -($C_0$-$C_6$)-alkyl-C(O)-NH-CN,
3) -O-($C_0$-$C_6$)-alkyl-C(O)-N(R9)-(R10), où R9 et R10 présentent la signification susmentionnée,
4) -($C_0$-$C_6$)-alkyl-C(O)-N(R8)-($C_0$-$C_6$)-alkyl-N(R9)-(R10),
où R8

i) signifie un atome d'hydrogène
ii) signifie -($C_1$-$C_6$)-alkyle, où alkyle est non substitué ou monosubstitué, disubstitué ou trisubstitué, indépendamment l'un de l'autre, par -$NH_2$, -CN, -OH, -C (O) -OH, -C (O) -NH-OH, $NO_2$, -C (O)-O-($C_1$-$C_6$)-alkyle ou halogène ou
iii) signifie -OH et

où R9 et R10 présentent la signification susmentionnée,
5) -($C_0$-$C_6$)-alkyl-C(O)-N(R8)-($C_0$-$C_6$)-alkyl-Het, où R8 a la signification susmentionnée et Het représente un système cyclique hétérocyclique saturé ou insaturé, monocyclique ou bicyclique, de 3 à 10 chaînons, qui contient 1, 2 ou 3 hétéroatomes de cycle identiques ou différents de la série azote, oxygène et soufre et qui peut être non substitué ou monosubstitué, disubstitué ou trisubstitué, indépendamment l'un de l'autre, par

a) halogène,
b) cyano,
c) nitro,
d) hydroxy,
e) amino,
f) -C(O)-O-($C_1$-$C_6$)-alkyle,
g) -C(O)-OH,
h) -($C_1$-$C_6$)-alkyle, où alkyle est non substitué ou monosubstitué, disubstitué ou trisubstitué par halogène,
i) -O-($C_1$-$C_6$)-alkyle, où alkyle est non substitué ou monosubstitué, disubstitué ou trisubstitué par halogène ou -N(R9)-(R10),
j) =O
k) -Het,
l) -($C_2$-$C_6$)-alcényle, où alcényle est non substitué ou monosubstitué, disubstitué ou trisubstitué par halogène ou -N(R9)-(R10), ou
m) -($C_2$-$C_6$)-alcynyle, où alcynyle est non substitué ou monosubstitué, disubstitué ou trisubstitué par halogène ou -N(R9)-(R10), ou

6) -($C_0$-$C_6$)-alkyl-C(O)-N(R8)-($C_0$-$C_6$)alkyl-($C_6$-$C_{14}$)-aryle, où aryle est non substitué ou monosubstitué, disubstitué ou trisubstitué, indépendamment l'un de l'autre, par les radicaux a) à m) susmentionnés,

R3, R4, R5, R6 et R7 sont identiques ou différents et représentent, indépendamment l'un de l'autre,

1. un atome d'hydrogène
2. halogène,
3. -($C_1$-$C_6$)-alkyle, où alkyle est non substitué ou monosubstitué, disubstitué ou trisubstitué par halogène,
4. -O-($C_1$-$C_6$)-alkyle, où alkyle est non substitué ou monosubstitué, disubstitué ou trisubstitué par halogène, ou
5. -S-($C_1$-$C_6$)-alkyle ou

R4 et R5 ou R5 et R6 forment ensemble avec les atomes de carbone auxquels ils sont à chaque fois liés, indépendamment l'un de l'autre, un cycle de 5 ou 6 chaînons, qui est aromatique ou saturé et qui contient zéro, un ou deux hétéroatomes de la série oxygène, azote et soufre, le cycle étant non substitué ou monosubstitué ou disubstitué sur un ou plusieurs atomes de carbone, par halogène et les autres radicaux R3, R6, et R7 ou R3, R4 et R7 ayant la signification susmentionnée de 1. à 5.

**2.** Composé de formule I selon la revendication 1, où

R1 représente un atome d'hydrogène ou -($C_1$-$C_6$)-alkyle,
R2 représente -($C_1$-$C_6$)-alkyle, où alkyle est monosubstitué, disubstitué ou trisubstitué par phényle, où phényle est monosubstitué, disubstitué ou trisubstitué, indépendamment l'un de l'autre, par

1) -($C_0$-$C_6$)-alkyl-C(O)-N(R9)-(R10), où R9 et R10 sont identiques ou différents et signifient, indépendamment l'un de l'autre

i) un atome d'hydrogène ou
ii) -($C_1$-$C_6$)-alkyle ou

R9 et R10 forment ensemble avec l'atome d'azote auquel ils sont liés, un cycle saturé de 5, 6 ou 7 chaînons, où un ou deux atomes de carbone peuvent également être remplacés par un hétéroatome de la série oxygène, soufre et azote, et, dans le cas de l'azote, les atomes d'azote peuvent être non substitués ou être substitués, indépendamment les uns des autres, par ($C_1$-$C_6$) -alkyle,

2) -($C_0$-$C_6$)-alkyl-C(O)-NH-CN,
3) -O-($C_0$-$C_6$)-alkyl-C(O)-N(R9)-(R10), où R9 et R10 présentent la signification susmentionnée,
4) -($C_0$-$C_6$)-alkyl-C(O)-N(R8)-($C_0$-$C_6$)-alkyl-N(R9)-(R10), où R8 signifie

i) un atome d'hydrogène
ii) -($C_1$-$C_6$)-alkyle, où alkyle est non substitué ou monosubstitué, disubstitué ou trisubstitué, indépendamment l'un de l'autre, par -$NH_2$, -CN, -OH, -C(O)-OH,- C(O)-NH-OH, $NO_2$, -C(O)-O-($C_1$-$C_6$)-alkyle ou halogène ou
iii) -OH et

où R9 et R10 présentent la signification susmentionnée,
5) -($C_0$-$C_6$)-alkyl-C(O)-N(R8)-($C_0$-$C_6$)-alkyl-Het, où R8 a la signification susmentionnée et Het représente un radical du groupe formé par les radicaux azépine, azétidine, aziridine, benzimidazole, benzofuranne, benzo[1,4]dioxine, 1,3-benzodioxol, 4H-benzo[1,4]oxazine, benzoxazole, benzothiazole, benzothiophène, quinazoline, quinoléine, quinoxaline, chromane, cinnoline, 1,2-diazépine, 1,3-diazépine, 1,4-diazépine, 1,4-dioxine, dioxole, furanne, imidazole, indazole, indole, isoquinoléine, isochromane, iso-indole, isothiazole, isoxazole, morpholine, 1,2-oxazine, 1,3-oxazine, 1,4-oxazine, oxazole, oxirane, pipérazine, pipéridine, phtalazine, pyranne, pyrazine, pyrazole, pyridazine, pyridine, pyrimidine, pyrido-imidazole, pyridopyridine, pyridopyrimidine, pyrrole, pyrrolidine, tétrazole, 1,2-thiazine, 1,3-thiazine, 1,4-thiazine, thiazole, thiomorpholine, thiophène, thiopyranne, 1,2,3-triazine, 1,3,5-triazine, 1,2,4-triazine, 1,2,3-triazole ou 1,2,4-triazole et où Het est non substitué ou est monosubstitué, disubstitué ou trisubstitué, indépendamment l'un de l'autre par

a) halogène,
b) cyano,
c) nitro,
d) hydroxy,
e) amino,
f) -C(O)-O-($C_1$-$C_6$)-alkyle,
g) -C(O)-OH,
h) -($C_1$-$C_6$)-alkyle, où alkyle est non substitué ou monosubstitué, disubstitué ou trisubstitué par halogène
i) -O-($C_1$-$C_6$)-alkyle, où alkyle est non substitué ou monosubstitué, disubstitué ou trisubstitué par halogène ou -N(R9)-(R10),
j) =O
k) -Het, où Het est défini comme ci-dessus,
l) -($C_2$-$C_6$)-alcényle, où alcényle est non substitué ou monosubstitué, disubstitué ou trisubstitué par halogène ou -N(R9)-(R10), ou
m) -($C_2$-$C_6$)-alcynyle, où alcynyle est non substitué ou monosubstitué, disubstitué ou trisubstitué par halogène ou -N(R9)-(R10), ou

6) -($C_0$-$C_6$)-alkyl-C(O)-N(R8)-($C_0$-$C_6$)-alkyl-($C_6$-$C_{14}$)-phényle,

où phényle est non substitué ou monosubstitué, disubstitué ou trisubstitué, indépendamment l'un de l'autre, par les radicaux a) à m) susmentionnés,

R3, R4, R5, R6 et R7 sont identiques ou différents et représentent, indépendamment l'un de l'autre,

1. un atome d'hydrogène,
2. halogène,
3. -($C_1$-$C_6$)-alkyle, où alkyle est non substitué ou monosubstitué, disubstitué ou trisubstitué par halogène, ou
4. -O-($C_1$-$C_6$)-alkyle, où alkyle est non substitué ou monosubstitué, disubstitué ou trisubstitué par halogène, ou

R4 et R5 ou R5 et R6 forment ensemble avec les atomes de carbone auxquels ils sont à chaque fois liés, indépendamment l'un de l'autre, un cycle dioxane, dioxole, dihydrofuranne ou furanne, le cycle étant non substitué ou monosubstitué ou disubstitué sur un ou plusieurs atomes de carbone, par halogène et les autres radicaux R3, R6 et R7 ou R3, R4 et R7 ayant la signification susmentionnée de 1. à 4.

**3.** Composé de formule I selon les revendications 1 ou 2, où

R1 représente un atome d'hydrogène,
R2 représente -($C_1$-$C_3$)-alkyle, où alkyle est substitué par phényle, où phényle est monosubstitué, disubstitué ou trisubstitué, indépendamment l'un de l'autre, par

1) -($C_0$-$C_6$)-alkyl-C(O)-N(R9)-(R10), où R9 et R10 représentent un atome d'hydrogène, méthyle, éthyle, propyle ou butyle, ou

R9 et R10 forment ensemble avec l'atome d'azote auquel ils sont liés un radical qui peut être dérivé de pyrrolidine, pipéridine, pyrazolidine, pyrazine, tétrazine, imidazolidine, pipérazine, isoxazolidine, morpholine, isothiazolidine ou thiomorpholine et, dans le cas de l'azote, les atomes d'azote peuvent, indépendamment l'un de l'autre, être non substitués ou substitués par ($C_1$-$C_4$) -alkyle,

2) -($C_0$-$C_4$)-alkyl-C(O)-NH-CN,
3) -O-($C_0$-$C_6$)-alkyl-C(O)-N(R9)-(R10), où R9 et R10 présentent la signification susmentionnée,
4) -($C_0$-$C_6$)-alkyl-C(O)-N(R8)-($C_0$-$C_6$)-alkyl-N(R9)-(R10), où R8 représente hydrogène, méthyle, éthyle, propyle ou butyle et R9 et R10 ont la signification susmentionnée
5) -C(O-(R8)-($C_0$-$C_2$)-alkyl-Het, où R8 a la signification susmentionnée et Het représente azépine, azétidine, aziridine, benzimidazole, benzofuranne, benzo[1,4]dioxine, 1,3-benzodioxole, 4H-benzo[1,4]oxazine, benzoxazole, benzothiazole, benzothiophène, quinazoline, quinoléine, quinoxaline, chromane, cinnoline, 1,2-diazépine, 1,3-diazépine, 1,4-diazépine, 1,4-dioxine, dioxole, furanne, imidazole, indazole, indole, isoquinoléine, isochromane, isoindole, isothiazole, isoxazole, morpholine, 1,2-oxazine, 1,3-oxazine, 1,4-oxazine, oxazole, oxirane, pipérazine, pipéridine, phtalazine, pyranne, pyrazine, pyrazole, pyridazine, pyridine, pyrimidine, pyridoimidazole, pyridopyridine, pyridopyrimidine, pyrrole, pyrrolidine, tétrazole, 1,2-thiazine, 1,3-thiazine, 1,4-thiazine, thiazole, thiomorpholine, thiophène, thiopyranne, 1,2,3-triazine, 1,3,5-triazine, 1,2,4-triazine, 1,2,3-triazole ou 1,2,4-triazole et où Het est non substitué ou monosubstitué, disubstitué ou trisubstitué, indépendamment l'un de l'autre par

a) halogène,
b) cyano,
c) nitro,
d) hydroxy,
e) amino,
f) -C(O)-O-($C_1$-$C_4$)-alkyle,
g) -C(O)-OH,
h) -($C_1$-$C_4$)-alkyle, où alkyle est non substitué ou monosubstitué, disubstitué ou trisubstitué par halogène
i) -O-($C_1$-$C_4$)-alkyle, où alkyle est non substitué ou monosubstitué, disubstitué ou trisubstitué par halogène, ou

6) -C(O)-N(R8)-($C_0$-$C_4$)-alkylphényle, où phényle est non substitué ou monosubstitué, disubstitué ou trisubstitué, indépendamment l'un de l'autre, par les radicaux susmentionnés a) à i),

R3, R4, R5, R6 et R7 sont identiques ou différents et représentent, indépendamment l'un de l'autre,

1. un atome d'hydrogène
2. halogène,
3. -($C_1$-$C_6$)-alkyle, où alkyle est non substitué ou monosubstitué, disubstitué ou trisubstitué par halogène,
4. -O-($C_1$-$C_6$)-alkyle, où alkyle est non substitué ou monosubstitué, disubstitué ou trisubstitué par halogène, ou

R4 et R5 ou R5 et R6 forment ensemble avec les atomes de carbone auxquels ils sont liés, indépendamment l'un de l'autre, un cycle dioxane, dioxole, dihydrofuranne ou furanne et les autres radicaux R3, R6 et R7 ou R3, R4 et R7 ayant la signification susmentionnée de 1. à 4.

**4.** Composé de formule I selon l'une ou plusieurs des revendications 1 à 3, un des composés suivants étant choisi :

4-(4-diéthylcarbamoylbenzylamide) 6-(3-méthoxybenzylamide) de l'acide pyrimidine-4,6-dicarboxylique,
4-(3-méthoxybenzylamide) 6-(4-propylcarbamoylbenzylamide) de l'acide pyrimidine-4,6-dicarboxylique,
4-(4-isopropylcarbamoylbenzylamide) 6-(3-méthoxybenzylamide) de l'acide pyrimidine-4,6-dicarboxylique,
4-[4-(2-diméthylaminoéthylcarbamoyl)-benzylamide] 6-(3-méthoxybenzylamide) de l'acide pyrimidine-4,6-dicarboxylique,
4-[(2,3-dihydrobenzo[1,4]dioxin-6-ylméthyl)-amide] 6-[4-(2-diméthylaminoéthylcarbamoyl)-benzylamide] de l'acide pyrimidine-4,6-dicarboxylique,
4-(3-chloro-4-fluorobenzylamide) 6-[4-(2-diméthylaminoéthylcarbamoyl)-benzylamide] de l'acide pyrimidine-4,6-dicarboxylique,
4-(4-fluoro-3-méthylbenzylamide) 6-{4-[2-(4-méthylpipérazin-1-yl)-2-oxoéthyl]-benzylamide} de l'acide pyrimidine-4,6-dicarboxylique,
4-(4-fluoro-3-méthylbenzylamide) 6-[4-(2-morpholin-4-yl-2-oxoéthoxy)-benzylamide] de l'acide pyrimidine-4,6-dicarboxylique,
4-(4-diéthylcarbamoylméthoxybenzylamide) 6-(4-fluoro-3-méthylbenzylamide) de l'acide pyrimidine-4,6-dicarboxylique,
4-(4-fluoro-3-méthylbenzylamide) 6-[4-(isopropylcarbamoylméthyl)-benzylamide] de l'acide pyrimidine-4,6-dicarboxylique,
4-(4-fluoro-3-méthylbenzylamide) 6-{4-[(2-morpholin-4-yléthylcarbamoyl)-méthyl]-benzylamide} de l'acide pyrimidine-4,6-dicarboxylique,
4-(4-diéthylcarbamoylméthylbenzylamide) 6-(4-fluoro-3-méthylbenzylamide) de l'acide pyrimidine-4,6-dicarboxylique,
4-(4-fluoro-3-méthylbenzylamide) 6-[4-(2-morpholin-4-yl-2-oxoéthyl)-benzylamide] de l'acide pyrimidine-4,6-dicarboxylique,
4-(4-fluoro-3-méthylbenzylamide) 6-[4-(isopropylcarbamoylméthoxy)-benzylamide] de l'acide pyrimidine-4,6-dicarboxylique,
4-(3-méthoxybenzylamide) 6-[4-(2-morpholin-4-yléthylcarbamoyl)-benzylamide] de l'acide pyrimidine-4,6-dicarboxylique,
4-(3-méthoxybenzylamide) 6-[4-(2-pyrrolidin-1-yléthylcarbamoyl)-benzylamide] de l'acide pyrimidine-4,6-dicarboxylique,
4-(4-fluoro-3-méthylbenzylamide) 6-[4-(thiomorpholine-4-carbonyl)-benzylamide] de l'acide pyrimidine-4,6-dicarboxylique,
4-(3-méthoxybenzylamide) 6-[4-(thiomorpholine-4-carbonyl)-benzylamide] de l'acide pyrimidine-4,6-dicarboxylique,
4-(4-cyanocarbamoylbenzylamide) 6-(4-fluoro-3-méthylbenzylamide) de l'acide pyrimidine-4,6-dicarboxylique,
4-(3-méthoxybenzylamide) 6-[4-(3-morpholin-4-ylpropylcarbamoyl)-benzylamide] de l'acide pyrimidine-4,6-dicarboxylique,
4-[(2,3-dihydrobenzofurann-5-ylméthyl)-amide] 6-[4-(3-morpholin-4-ylpropylcarbamoyl)-benzylamide] de l'acide pyrimidine-4,6-dicarboxylique,
4-(4-fluoro-3-méthylbenzylamide) 6-[4-(4-méthylpipérazine-1-carbonyl)-benzylamide] de l'acide pyrimidine-4,6-dicarboxylique,
4-[(2,3-dihydrobenzofurann-5-ylméthyl)-amide] 6-{4-[(pyridin-4-ylméthyl)-carbamoyl]-benzylamide} de l'acide pyrimidine-4,6-dicarboxylique,
4-(4-N-cyanocarbamoylbenzylamide) 6-[(2,3-dihydrobenzfurann-5-ylméthyl)-amide] de l'acide pyrimidine-4,6-dicarboxylique,

4-(4-N-cyanocarbamoylbenzylamide) 6-(3-méthoxybenzylamide) de l'acide pyrimidine-4,6-dicarboxylique,
4-(4-fluoro-3-méthylbenzylamide) 6-[4-(morpholine-4-carbonyl)-benzylamide] de l'acide pyrimidine-4,6-dicarboxylique,
4-(4-fluoro-3-méthylbenzylamide) 6-[4-(2-pipérazin-1-yléthylcarbamoyl)-benzylamide] de l'acide pyrimidine-4,6-dicarboxylique,
4-(4-tert-butylcarbamoylbenzylamide) 6-(3-méthoxybenzylamide) de l'acide pyrimidine-4,6-dicarboxylique,
4-(3-méthoxybenzylamide) 6-{4-[méthyl-(1-méthylpipéridin-4-yl)-carbamoyl]-benzylamide} de l'acide pyrimidine-4,6-dicarboxylique,
4-(4-fluoro-3-méthylbenzylamide) 6-[4-(2-pyrrolidin-1-yléthylcarbamoyl)-benzylamide] de l'acide pyrimidine-4,6-dicarboxylique,
4-(3-méthoxybenzylamide) 6-[3-(2-morpholin-4-yléthylcarbamoyl)-benzylamide] de l'acide pyrimidine-4,6-dicarboxylique,
4-(4-fluoro-3-méthylbenzylamide) 6-[4-(2-morpholin-4-yléthylcarbamoyl)-benzylamide] de l'acide pyrimidine-4,6-dicarboxylique,
ester méthylique de l'acide [4-({[6-(4-fluoro-3-méthylbenzylcarbamoyl)-pyrimidine-4-carbonyl]-amino}-méthyl)-benzoylamino]-acétique,
4-(3-méthoxybenzylamide) 6-[3-(morpholine-4-carbonyl)-benzylamide] de l'acide pyrimidine-4,6-dicarboxylique,
4-(3-méthoxybenzylamide) 6-{4-[(pipéridin-4-ylméthyl)carbamoyl]-benzylamide} de l'acide pyrimidine-4,6-dicarboxylique,
4-(3-méthoxybenzylamide) 6-[4-(pipéridin-4-ylcarbamoyl)-benzylamide] de l'acide pyrimidine-4,6-dicarboxylique,
4-(4-fluoro-3-méthylbenzylamide) 6-[4-(pipéridin-4-ylcarbamoyl)-benzylamide] de l'acide pyrimidine-4,6-dicarboxylique,
4-(4-fluoro-3-méthylbenzylamide) 6-{4-[méthyl-(1-méthylpipéridin-4-yl)-carbamoyl]-benzylamide} de l'acide pyrimidine-4,6-dicarboxylique,
4-(4-fluoro-3-méthylbenzylamide) 6-{4-[(pipéridin-4-ylméthyl)-carbamoyl]-benzylamide} de l'acide pyrimidine-4,6-dicarboxylique,
4-(3-méthoxybenzylamide) 6-[4-(4-méthylpipérazine-1-carbonyl)-benzylamide] de l'acide pyrimidine-4,6-dicarboxylique,
4-[(2,3-dihydrobenzofurann-5-ylméthyl)-amide] 6-[4-(morpholin-4-carbonyl)-benzylamide] de l'acide pyrimidine-4,6-dicarboxylique,
4-[(2,3-dihydrobenzofurann-5-ylméthyl)-amide] 6-[4-(4-méthylpipérazin-1-carbonyl)-benzylamide] de l'acide pyrimidine-4,6-dicarboxylique,
4-[(2,3-dihydrobenzofurann-5-ylméthyl)-amide] 6-[4-(2-pyrrolidin-1-yléthylcarbamoyl)-benzylamide] de l'acide pyrimidine-4,6-dicarboxylique,
4-[(2,3-dihydrobenzofurann-5-ylméthyl)-amide] 6-[4-(2-morpholin-4-yléthylcarbamoyl)-benzylamide] de l'acide pyrimidine-4,6-dicarboxylique,
4-(3-chloro-4-fluorobenzylamide) 6-[4-(2-pyrrolidin-1-yléthylcarbamoyl)-benzylamide] de l'acide pyrimidine-4,6-dicarboxylique,
4-(3-méthoxybenzylamide) 6-[4-(morpholine-4-carbonyl)-benzylamide] de l'acide pyrimidine-4,6-dicarboxylique,
4-(3-méthoxybenzylamide) 6-{4-[(pyridin-4-ylméthyl)-carbamoyl]-benzylamide} de l'acide pyrimidine-4,6-dicarboxylique,
4-(3-chloro-4-fluorobenzylamide) 6-(4-diéthylcarbamoylbenzylamide) de l'acide pyrimidine-4,6-dicarboxylique,
4-(3-chloro-4-fluorobenzylamide) 6-[4-(morpholine-4-carbonyl)-benzylamide] de l'acide pyrimidine-4,6-dicarboxylique,
4-(3-chloro-4-fluorobenzylamide) 6-[4-(2-morpholin-4-yléthylcarbamoyl)-benzylamide] ou
4-(4-diéthylcarbamoylbenzylamide) 6-(3-méthoxybenzylamide) de l'acide pyrimidine-4,6-dicarboxylique.

**5.** Procédé pour la préparation du composé de formule I selon l'une ou plusieurs des revendications 1 à 4, **caractérisé en ce qu'**on transforme un composé de formule II

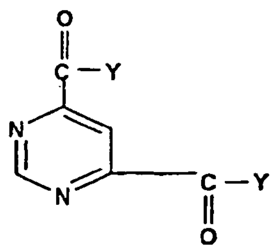

( II )

a) avec un composé de formule IIIa ou IIIb

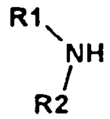

IIIa

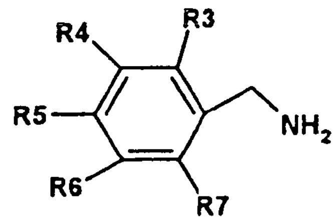

IIIb

où R1, R2, R3, R4, R5, R6 et R7 ont les significations indiquées dans la formule I et Y représente halogène, hydroxyle ou $C_1$-$C_4$-alcoxy ou forme, ensemble avec le groupe carbonyle, un ester actif ou un anhydride mixte, en formant un composé de formule I et on transforme le cas échéant les produits de réaction en leurs sels physiologiquement acceptables, ou

b) on transforme un composé de formule II avec un composé de formule IIIa ou IIIb en un composé de formule IVa ou IVb

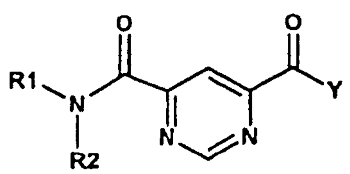

(IVa)

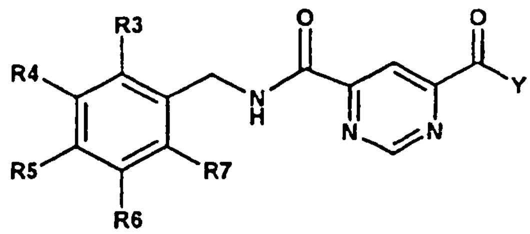

(IVb)

où R1 à R7 ont les significations indiquées dans la formule I et Y représente halogène, hydroxyle ou $C_1$-$C_4$-alcoxy ou forme, ensemble avec le groupe carbonyle, un ester actif ou un anhydride mixte, on purifie le cas échéant le composé de formule IVa ou IVb et on transforme ensuite avec un composé de formule IIIa ou IIIb en un composé de formule I.

6. Médicament, **caractérisé par** une teneur active en au moins un composé de formule I selon l'une ou plusieurs des revendications 1 à 4 avec un support, un additif pharmaceutiquement approprié et physiologiquement compatible et/ou d'autres substances actives et adjuvants.

7. Utilisation du composé de formule I selon l'une ou plusieurs des revendications 1 à 4 pour la préparation d'un médicament destiné à la prophylaxie et à la thérapie de maladies dégénératives des articulations, telles que les ostéoarthroses, les spondyloses, une atrophie des cartilages après un traumatisme articulaire ou un repos prolongé des articulations après des lésions au ménisque ou à la rotule ou des déchirures de ligaments, comprenant également les maladies du tissu conjonctif, telles que les collagénoses, les maladies du périodonte, les troubles de guérison de plaies et les maladies chroniques de l'appareil locomoteur, telles que les arthrites aiguës et chroniques, inflammatoires, immunologiques ou dues au métabolisme, les arthropathies, les myalgies et les troubles du métabolisme des os ou les maladies cancéreuses, telles que le cancer du sein.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0418797 A
- EP 0463592 A
- WO 02064571 A
- WO 02064080 A
- WO 03049738 A
- EP 0606046 A
- WO 9428889 A


### In der Beschreibung aufgeführte Nicht-Patentliteratur


- **MASSOVA I. et al.** *The FASEB Journal,* 1998, vol. 12, 1075-1095
- **WEITHMANN et al.** *Inflamm Res,* 1997, vol. 46, 246-252